# EUROPEAN PATENT SPECIFICATION

(11) **EP 2 742 033 B1**
(45) Date of publication and mention of the grant of the patent: **19.09.2018**
(21) Application number: 12821405.3
(22) Date of filing: 13.08.2012
(51) Int. Cl.: C07D 309/32, C07D 215/233, C07D 335/06

(54) **FLAVONOID COMPOUNDS, AND METHODS OF USE THEREOF**
FLAVONOIDVERBINDUNGEN UND ANWENDUNGSVERFAHREN DAFÜR
COMPOSÉS FLAVONOÏDES ET LEURS PROCÉDÉS D'UTILISATION

(30) Priority: 11.08.2011 AU 2011903218
(43) Date of publication of application: 18.06.2014
(73) Proprietor: ARMARON BIO PTY LTD, East Melbourne, VIC 3002 (AU)
(72) Inventor: MCLACHLAN, Grant, Melbourne, Victoria 3128 (AU)
(74) Representative: Abel & Imray
(86) International application number: PCT/AU2012/000953
(87) International publication number: WO 2013/020184

(56) References cited:
- WO-A1-00/12496
- WO-A1-01/83469
- WO-A1-2009/102498
- WO-A1-2009/109230
- WO-A2-01/81340
- AU-A1- 2006 233 256
- AU-A1- 2006 233 256
- JP-A- 2003 313 182
- JP-A- 2007 051 099
- SUWAN YAP ET AL: "Synthesis of a hypoxia-targeted conjugate of the cardioprotective agent 3,4-dihydroxyflavonol and evaluation of its ability to reduce ischaemia/reperfusion injury", BIOORGANIC & MEDICINAL CHEMISTRY LETTERS, PERGAMON, AMSTERDAM, NL, vol. 21, no. 17, 10 March 2011 (2011-03-10), pages 5102-5106, XP028263975, ISSN: 0960-894X, DOI: 10.1016/J.BMCL.2011.03.040 [retrieved on 2011-03-17]
- VERMA ET AL: "Understanding topoisomerase I and II in terms of QSAR", BIOORGANIC & MEDICINAL CHEMISTRY, PERGAMON, GB, vol. 13, no. 4, 15 February 2005 (2005-02-15), pages 1059-1067, XP027637767, ISSN: 0968-0896 [retrieved on 2005-02-15]
- SUI Z ET AL: "Synthesis and topoisomerase inhibitory activities of novel aza-analogues of flavones", EUROPEAN JOURNAL OF MEDICINAL CHEMISTRY, EDITIONS SCIENTIFIQUE ELSEVIER, PARIS, FR, vol. 34, no. 5, 1 May 1999 (1999-05-01), pages 381-387, XP004180345, ISSN: 0223-5234, DOI: 10.1016/S0223-5234(99)80087-7
- YAP, S. ET AL.: 'Synthesis of a hypoxia-targeted conjugate of the cardioprotective agent 3',4'-dihydroxyflavonol and evaluation of its ability to reduce ischaemia/reperfusion injury' BIOORGANIC & MEDICINAL CHEMISTRY LETTERS vol. 21, 17 March 2011, pages 5102 - 5106, XP028263975
- LEE, J. ET AL.: '3-Hydroxychromones as cyclin-dependent kinase inhibitors: Synthesis and biological evaluation' BIOORGANIC & MEDICINAL CHEMISTRY LETTERS vol. 17, 2007, pages 1284 - 1287, XP005888465
- MATTAREI, A ET AL.: 'Regioselective O-Derivatization of Quercetin via Ester Intermediates. An Improved Synthesis of Rhamnetin and Development of a New Mitochondriotropic Derivative' MOLECULES vol. 15, 2010, pages 4722 - 4736, XP002635413
- LI, N. ET AL.: 'Two new flavonoid alkaloids from Senecio argunensis' JOURNAL OF ASIAN NATURAL PRODUCTS RESEARCH vol. 10, no. 12, 2008, pages 1143 - 1146, XP055144056
- HOWELL, H. ET AL.: 'Identification of Two Novel Promelacacinidin Dimers from Acacia nigrescens' JOURNAL OF NATURAL PRODUCTS vol. 65, no. 5, 2002, pages 769 - 771, XP055144057
- ITO, C. ET AL.: 'A New Biflavonoid from Calophyllum panciflorum with Antitumor- Promoting Activity' JOURNAL OF NATURAL PRODUCTS vol. 62, no. 12, 1999, pages 1668 - 1671, XP055144060
- PICQ, M. ET AL.: 'Pentasubstituted Quercetin Analogues as Selective Inhibitors of Particulate 3':5'-Cyclic-AMP Phosphodiesterase from Rat Brain' JOURNAL OF MEDICINAL CHEMISTRY vol. 25, 1982, pages 1192 - 1198, XP055107500
- ENRIQUEZ, P. M.: 'Novel cobalt (II) complexes as catalysts for redox couple amplification processes' RESEARCH DISCLOSURE vol. 15541, March 1977, pages 93 - 94, XP007104601
- DATABASE REGISTRY [Online] 19 March 2010 XP003032597 Database accession no. 1211594-00-1
- DATABASE REGISTRY [Online] 19 March 2010 XP003032598 Database accession no. 1211593-18-8

## Description

### Cross-Reference to Related Applications

### Technical Field

The present invention relates to novel compounds, compositions containing these compounds, methods for their synthesis, and uses of these compounds. In particular, the present invention relates to novel flavonoid compounds, methods of synthesizing the flavonoid compounds, compositions containing the flavonoid compounds and methods of their use.

### Background

Apoptosis is a form of programmed cell death. Apoptosis occurs in the acute phase of ischaemia / reperfusion injury, such as in stroke and acute myocardial infarction. It also occurs in the non-infarcted heart chronically post acute myocardial infarction, and diabetes and various other diseases. One of the key stressors that drives apoptosis is oxidative stress.

Post reperfusion injury, tissue remodelling occurs. Indeed, acute myocardial infarction is the leading cause of chronic heart failure. The injury caused by ischaemia and reperfusion causes acute cardiomyocyte cell death in the area at risk. The loss of cardiac muscle affects the hearts function and the heart muscle remodels over a period of time to compensate. The mechanical stress in the post AMI heart leads to apoptosis of cardiomyocytes in the non-affected left ventricle. Reactive oxygen species are believed to be a key driver of the apoptotic process.

Elevated cellular apoptosis also occurs in diabetes where elevated glucose levels lead to high levels of oxidative stress, causing apoptosis in the diabetic cardiac tissue and also in the vasculature.

Apoptosis is controlled by Mitogen-Activated Protein Kinases (MAPK), activities, including Jun N-terminal kinases (JNK), p38α, and extracellular-signal-regulated kinases (ERK).

The activities of these kinases are markedly modulated in response to cellular stressors, such as during ischaemia and post reperfusion of tissue, in the chronic phase of cardiac remodelling in diabetes and neurodegenerative conditions. JNK and p38α activation, which are also referred to collectively as stress activated protein kinases (SAPK), have been found to have a pro-apoptotic effect while ERK activation has an anti-apoptotic effect. The relative extent of JNK, p38α, or ERK activation has been proposed to determine cell fate after injury.

One approach for preventing and / or mitigating the damage caused by apoptosis and / or cell necrosis has been to administer compounds that inhibit the pro- apoptotic signalling pathways, p38α and JNK. Another approach would be to activate the anti-apoptotic ERK signalling pathway. A molecule that had both properties may have an even greater therapeutic benefit.

One approach for preventing and / or mitigating the damage caused by apoptosis may be to administer flavonoid compounds. The basic structure of flavonoids is the following:

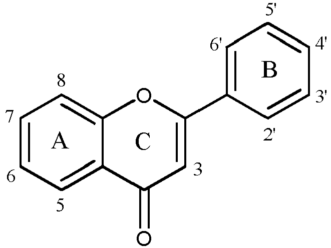

For example, the synthetic flavonoid, 3',4'-dihydroxy flavonol (DiOHF) has been demonstrated to reduce infarct and injury associated with myocardial ischaemia and reperfusion during *in vitro* studies (Shen Wang, Gregory Dusting, Clive May and Owen Woodman, British Journal of Pharmacology (2004) 142, 443-452). Synthesis and evaluation of a hypoxia-targeted conjugate of DiOHF has been disclosed (S.Yap et al., Bioorganic & Medicinal Chemistry Letters, vol. 21, 2011, 5102-5106).

There is, however, a continuing need for alternative compounds that have good biological and pharmacokinetic properties.

### Summary

One aspect relates to a compound of the general formula (I): 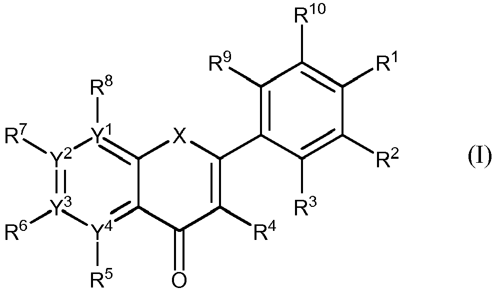 wherein
X represents an NH, S or O;
Y¹, Y², Y³, Y⁴ are independently selected from C or N;
R¹, R² and R⁴ are independently selected from the group comprising hydroxyl, haloalkyl, haloalkoxy, heterocyclic and substituted heterocyclic, -NH₂, -NH(C₁-C₆alkyl) and-N(C₁-C₆ alkyl)₂;
R³, R⁹ and R¹⁰ are independently selected from the group comprising H, halogen and C₁-C₆ alkyl;
R⁵, R⁶, R⁷ and R⁸ where present are H; or
a pharmaceutically acceptable salt, hydrate and/or solvate thereof;
with the *proviso* that at least one of R¹, R², and R⁴ is selected from the group comprising haloalkyl, haloalkoxy, heterocyclic, substituted heterocyclic, -NH₂, -NH(C₁-C₆ alkyl) and -N(C₁-C₆ alkyl)₂;
with the *proviso* that the compound is not selected from the group comprising:
   2-(3,4-dihydroxyphenyl)-3,5,7-trihydroxy-4H-1-benzothiopyran-4-one;
   2-(2,3-dihydroxyphenyl)-5,8-dihydroxy-4H-1-benzothiopyran-4-one;
   2-(3,4-dihydroxyphenyl)-3-hydroxy-4(1H)-quinolinone;
   2-(3,4-dihydroxyphenyl)-3,7-dihydroxy-4(1H)-quino linone, hydrobromide;
   2-(3,4-dihydroxyphenyl)-3,7-dihydroxy-4(1H)-quinolinone;
   2-(3,4-dihydroxyphenyl)-3,6-dihydroxy-4(1H)-quinolinone;
   2-(3,4-dihydroxyphenyl)-3,5-dihydroxy-4(1H)-quinolinone, hydrobromide;
   2-(3,4-dihydroxyphenyl)-3,5-dihydroxy-4(1H)-quinolinone;
   2-(3,4-dihydroxyphenyl)-3,6,7-trihydroxy-4(1H)-quinolinone;
   2-(3,4-dihydroxyphenyl)-3,5,7-trihydroxy-4(1H)-quino linone;
   2-(3,4-dihydroxyphenyl)-3-hydroxy-5,7-dimethyl-4(1H)-quino linone, hydrobromide;
   2-(3,4-dihydroxyphenyl)-3-hydroxy-5,7-dimethyl-4(1H)-quino linone;
   2-(3,4-dihydroxyphenyl)-3-hydroxy-7-methoxy-4(1H)-quinolinone;
   2-(3,4-dihydroxyphenyl)-3-hydroxy-6-methoxy-4(1H)-quinolinone;
   2-(3,4-dihydroxyphenyl)-3,6,7,8-tetrahydroxy-4(1H)-quinolinone;
   2-(3,4-dihydroxyphenyl)-3-hydroxy-5-methoxy-4(1H)-quinolinone;
   2-(3,4-dihydroxyphenyl)-3-hydroxy-6,7-dimethoxy-4(1H)-quinolinone;
   2-(3-amino-4-hydroxyphenyl)-3,5,7-trihydroxy-4H-1-benzopyran-4-one;
   N-[2-(3-amino-4-hydroxyphenyl)-3-hydroxy-6-methyl-4-oxo-4H-chromen-8-yl]-acetamide;
   N-[3-hydroxy-2-[4-hydroxy-3-[91-methylethyl)amino]phenyl]6-methyl-4-oxo-4H-1-benzopyran-8-yl]-acetamide;
   N-[2-hydroxy-5-(3-hydroxy-6-methyl-4-oxo-4H-1-benzopyran-2-yl)phenyl]-acetamide;
   2-(3-amino-4-hydroxyphenyl)-3-hydroxy-6-methyl-4H-1-benzopyran-4-one;
   8-amino-2-(3-amino-4-hydroxyphenyl)-3-hydroxy-6-methyl-4H-1-benzopyran-4-one;
   N-[2-Hydroxy-5-(3-hydroxy-6-methyl-4-oxo-4H-1-benzopyran-2-yl)phenyl]-acetamide;
   N-[2-(3,4-dihydroxyphenyl)-3-hydroxy-4-oxo-4H-1-benzopyran-6-yl]-acetamide;
   N-[2-(3-amino-4-hydroxyphenyl)-3-hydroxy-6-methyl-4-oxo-4H-1-benzopyran-8-yl]-acetamide;
   6-acetamido-3-hydroxy-4'-methoxy-flavone;
   3,3'-dihydroxy, 6,7-dimethyl, 4'-(pyrrolidine-1-yl)-flavone; and
   2-(3-amino-4-hydrophenyl)-3,5,7-trihydroxy-4H-1-benzopyran-4-one.

Another aspect relates to a compound of formula (I) for use in the prevention and/or mitigation of a disease caused by cell apoptosis and/or cell necrosis in a subject: 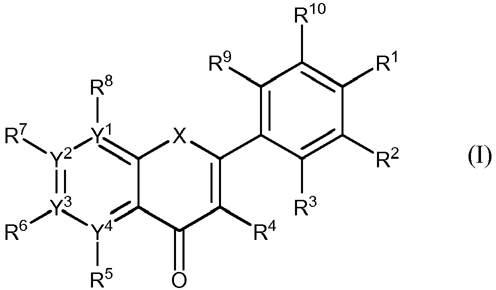 wherein
X represents an NH, S or O;
Y¹, Y², Y³, Y⁴ are independently selected from C or N;
R¹, R² and R⁴ are independently selected from the group comprising hydroxyl, haloalkyl, haloalkoxy, heterocyclic, substituted heterocyclic, -NH₂, -NH(C₁-C₆ alkyl) and - N(C₁-C₆ alkyl)₂;
R³, R⁹ and R¹⁰ are independently selected from the group comprising H, halogen and C₁-C₆ alkyl;
R⁵, R⁶, R⁷ and R⁸ where present are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and -N(1 C₁-C₆ alkyl)₂.

Another aspect relates to a compound of formula (I) for use in the prevention and /or mitigation of ischaemia, reperfusion injury, cerebrovascular disease, pulmonary vascular disease, atherosclerosis, stroke, myocardial infarction, artery disease, congestive heart disease, coronary disease, peripheral vascular disease, diabetes, hypertension, migraine, chronic obstructive pulmonary disease or retinal vascular disease: 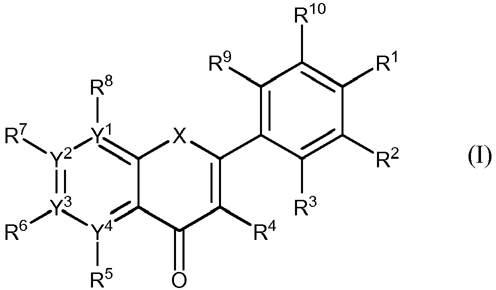 wherein
X represents an NH, S or O;
Y¹, Y², Y³, Y⁴ are independently selected from C or N;
R¹, R² and R⁴ are independently selected from the group comprising hydroxyl, haloalkyl, haloalkoxy, heterocyclic, substituted heterocyclic, -NH₂, -NH(C₁-C₆ alkyl) and-N(C₁-C₆ alkyl)₂;
R³, R⁹ and R¹⁰ are independently selected from the group comprising H, halogen and C₁-C₆ alkyl;
R⁵, R⁶, R⁷ and R⁸ where present are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and-N(C₁-C₆ alkyl)₂.

Another aspect relates to a compound of formula (I) for use in the prevention and/or mitigation of ischaemia or reperfusion injury: 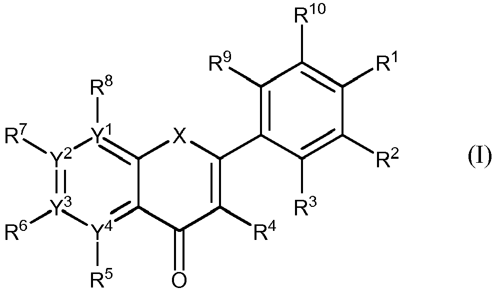 wherein
X represents an NH, S or O;
Y¹, Y², Y³, Y⁴ are independently selected from C or N;
R¹, R² and R⁴ are independently selected from the group comprising hydroxyl, haloalkyl, haloalkoxy, heterocyclic, substituted heterocyclic, -NH₂, -NH(C₁-C₆ alkyl) and-N(C₁-C₆ alkyl)₂;
R³, R⁹ and R¹⁰ are independently selected from the group comprising H, halogen and C₁-C₆ alkyl;
R⁵, R⁶, R⁷ and R⁸ where present are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and - N(C₁-C₆ alkyl)₂.

One aspect relates to a compound of formula (I) for use in ameliorating damage to a subject caused by the administration of a therapeutic agent: 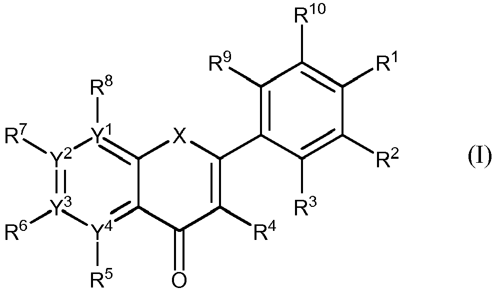 wherein
X represents an NH, S or O;
Y¹, Y², Y³, Y⁴ are independently selected from C or N;
R¹, R² and R⁴ are independently selected from the group comprising hydroxyl, haloalkyl, haloalkoxy, heterocyclic, substituted heterocyclic, -NH₂, -NH(C₁-C₆ alkyl) and - N(C₁-C₆ alkyl)₂;
R³, R⁹ and R¹⁰ are independently selected from the group comprising H, halogen and C₁-C₆ alkyl;
R⁵, R⁶, R⁷ and R⁸ where present are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆alkyl) and -N(C₁-C₆ alkyl)₂.

Another aspect relates to a compound of formula (I) for use in the prevention and/or treatment of a disease(s) in a subject associated with the presence of reactive oxidative species (ROS): 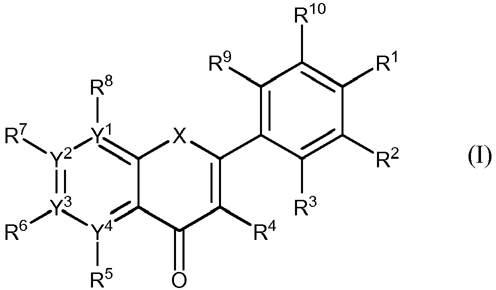 wherein
X represents an NH, S or O;
Y¹, Y², Y³, Y⁴ are independently selected from C or N;
R¹, R² and R⁴ are independently selected from the group comprising hydroxyl, haloalkyl, haloalkoxy, heterocyclic, substituted heterocyclic, -NH₂, -NH(C₁-C₆ alkyl) and - N(C₁-C₆ alkyl)₂;
R³, R⁹ and R¹⁰ are independently selected from the group comprising H, halogen and C₁-C₆ alkyl;
R⁵, R⁶, R⁷ and R⁸ where present are H; or
a pharmaceutically acceptable salt, hydrate and/or solvate thereof;
with the *proviso* that at least one of R¹, R², R⁴, R⁵, R⁶, R⁷ and R⁸ is selected from the group comprising haloalkyl, haloalkoxy, heterocyclic, substituted heterocyclic, -NH₂,-NH(C₁-C₆ alkyl) and -N(C₁-C₆ alkyl)₂.

The compound of formula (I) may be for use in the prevention and /or mitigation of a disease caused by cell apoptosis and / or cell necrosis in a subject, and/or for use in the prevention and/or treatment of a disease(s) in a subject associated with the presence of reactive oxidative species (ROS) for use in the treatment of reperfusion injury as a result of an acute or chronic condition, the subject suffering from mechanical trauma resulting from crush injury or surgery; ischaemia and/or reperfusion injury as a result of an acute or chronic condition; disease which is the result of vascular surgery or disease is the result of heart bypass and/or transplant surgery.

A method of inhibiting pro apoptotic signalling kinases is also described, the method comprising administering a therapeutically effective amount of at least one compound in accordance with formula (I).

A method of activating extracellular signal-regulated kinase (ERK) activity is also described, the method comprising administering a therapeutically effective amount of at least one compound in accordance with formula (I).

A method of inhibiting p38α and JNK activity while simultaneously activating ERK activity is also described, the method comprising administering a therapeutically effective amount of at least one compound in accordance with formula (I).

The haloalkyl may be a fluroalkyl. The fluoroalkyl may be selected from the group comprising -CF₂H, -CH₂F, -CH₂CF₂H, and -CF₂CF₂H. In certain aspects, the fluoroalkyl is-CF₂H.

The haloalkoxy may be selected from the group comprising -OCF₂H, -OCH₂F,-OCH₂CF₂H, -OCF₂CF₂H. In certain aspects, the haloalkyl is -OCF₂H.

The halogen may be an F, Cl, Br or I. In certain aspects, the halogen is F.

The heterocyclic may be a saturated or unsaturated group having a single ring or multiple condensed rings, from 1 to 10 ring carbon atoms and from 1 to 4 ring hetero atoms selected from nitrogen, sulfur or oxygen within the ring wherein, in fused ring systems, one or more of the rings can be aryl or heteroaryl. In certain aspects, the heterocyclic may be selected from the group comprising

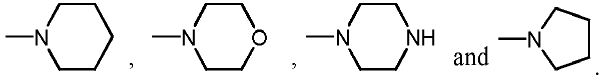

In a particular aspect, the heterocyclic is

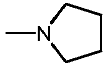

The -NH(C₁-C₆ alkyl) group may be selected from the group comprising -NHCH₃,-NHCH₂CH₃ and -NHCH₂CH₂CH₃. In certain aspects, the -NH(C₁-C₆ alkyl) is a -NHCH₃ group.

In one aspect, the -NH(C₁-C₆ alkyl) may be a substituted -NH(C₁-C₆ alkyl) group.

The N(C₁-C₆ alkyl)₂ may be a selected from -N(CH₃)₂, -N(CH₂CH₃)₂, and-N(CH₂CH₂CH₃)₂.

In one aspect, X is NH. In another aspect, X is a S. In yet another aspect, X is O.

In one aspect, Y¹, Y², Y³, Y⁴ are all C.

In some embodiments, R³, R⁹ and R¹⁰ are independently H or a halogen, in particular, a F.

A method of preventing and / or mitigating the damage caused by cell apoptosis or cell necrosis is also disclosed.

A method of activating extracellular-signal-regulated kinase (ERK) activity is also disclosed.

Without wishing to be bound by theory, it is thought that compounds of the present invention may act as both p38α and JNK inhibitor and an activator of ERK activity.

A method of inhibiting p38α and JNK activity while simultaneously activating ERK activity is also disclosed.

### Description of Embodiments

One aspect relates to a compound of formula (II): 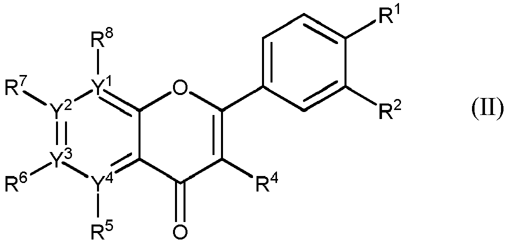 wherein
Y¹, Y², Y³, Y⁴ are independently selected from C or N;
R¹, R² and R⁴ are independently selected from the group comprising hydroxyl, haloalkyl, haloalkoxy, heterocyclic, substituted heterocyclic, -NH₂, -NH(C₁-C₆ alkyl) and-N(C₁-C₆ alkyl)₂;
R⁵, R⁶, R⁷ and R⁸ where present are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and -N(C₁-C₆ alkyl)₂.

One aspect relates to a compound of formula (IIa): 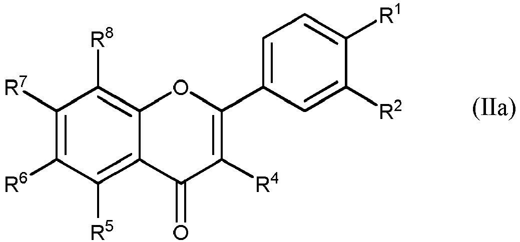 wherein
R¹, R² and R⁴ are independently selected from the group comprising hydroxyl, haloalkyl, haloalkoxy, heterocyclic, substituted heterocyclic, -NH₂, -NH(C₁-C₆ alkyl) and-N(C₁-C₆ alkyl)₂;
R⁵, R⁶, R⁷ and R⁸ are H; or
a pharmaceutically acceptable salt, hydrate and/orsolvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and-N(C₁-C₆ alkyl)₂.

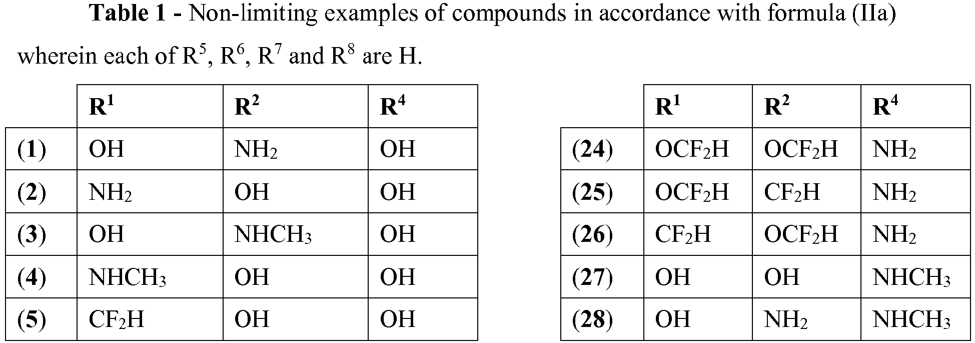

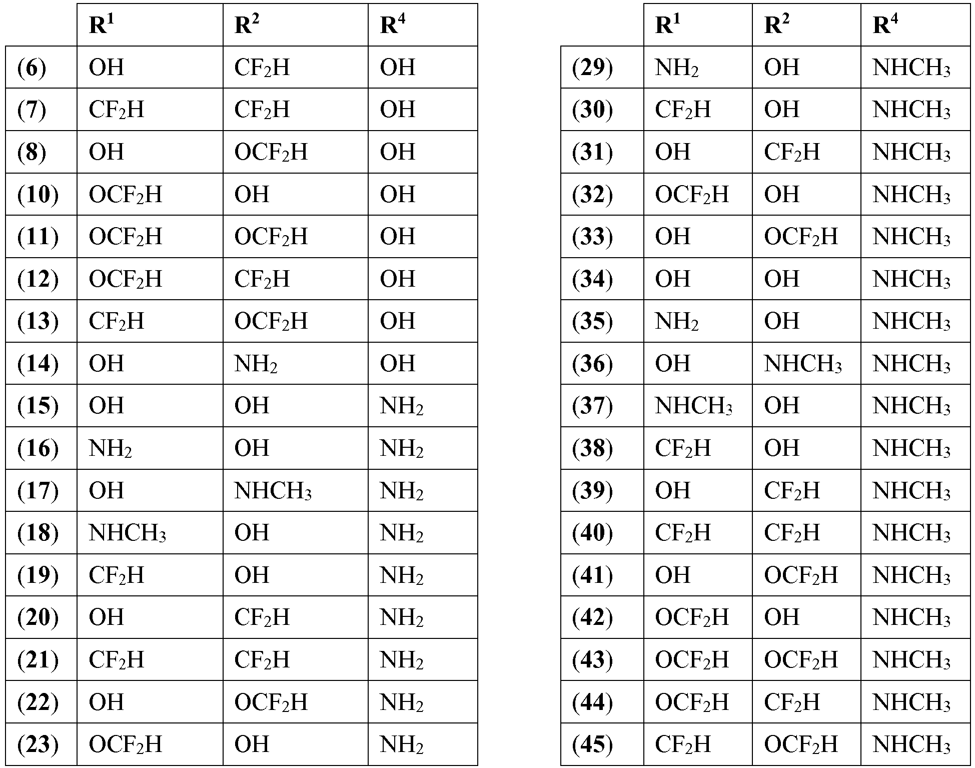

A compound of formula (IIaa) is also disclosed: 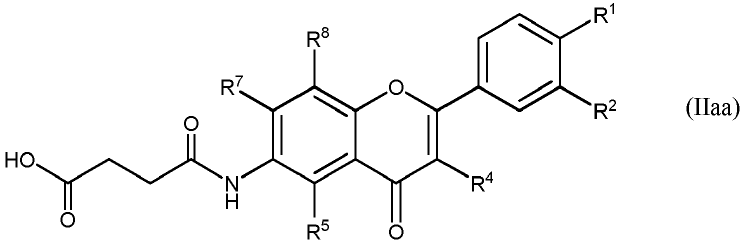 wherein
R¹, R² and R⁴ are independently selected from the group comprising hydroxyl, haloalkyl, haloalkoxy, heterocyclic, substituted heterocyclic, -NH₂, -NH(lower alkyl) and-N(lower alkyl)₂;
R⁵, R⁷ and R⁸ are independently selected from the group comprising H, lower alkyl, halogen, hydroxyl, ester, haloalkyl, haloalkoxy, lower alkoxy, heterocyclic, substituted heterocyclic, -NH₂, -NH(lower alkyl), and -N(lower alkyl)₂; or a pharmaceutically acceptable salt, hydrate, solvate, prodrug and isomer thereof;
with the *proviso* that at least one of R¹, R², R⁴, R⁵, R⁷ and R⁸ is selected from the group comprising haloalkyl, haloalkoxy, heterocyclic, substituted heterocyclic, -NH₂, -NH(lower alkyl) and -N(lower alkyl)₂.

**Table 2 - Non-limiting examples of compounds in accordance with formula (IIaa) wherein each of R⁵, R⁷ and R⁸ are H.**

| | **R¹** | **R²** | **R⁴** |
|---|---|---|---|
| **(46)** | OH | NH₂ | OH |
| **(47)** | NH₂ | OH | OH |
| **(48)** | OH | NHCH₃ | OH |
| **(49)** | NHCH₃ | OH | OH |
| **(50)** | CF₂H | OH | OH |
| **(51)** | OH | CF₂H | OH |
| **(52)** | CF₂H | CF₂H | OH |
| **(53)** | OH | OCF₂H | OH |
| **(54)** | OCF₂H | OH | OH |
| **(55)** | OCF₂H | OCF₂H | OH |
| **(56)** | OCF₂H | CF₂H | OH |
| **(57)** | CF₂H | OCF₂H | OH |
| **(58)** | OH | NH₂ | OH |
| **(59)** | OH | OH | NH₂ |
| **(60)** | NH₂ | OH | NH₂ |
| **(61)** | OH | NHCH₃ | NH₂ |
| **(62)** | NHCH₃ | OH | NH₂ |
| **(63)** | CF₂H | OH | NH₂ |
| **(64)** | OH | CF₂H | NH₂ |
| **(65)** | CF₂H | CF₂H | NH₂ |
| **(66)** | OH | OCF₂H | NH₂ |
| **(67)** | OCF₂H | OH | NH₂ |
| **(68)** | OCF₂H | OCF₂H | NH₂ |
| **(69)** | OCF₂H | CF₂H | NH₂ |
| **(70)** | CF₂H | OCF₂H | NH₂ |
| **(71)** | OH | OH | NHCH₃ |
| **(72)** | OH | NH₂ | NHCH₃ |
| **(73)** | NH₂ | OH | NHCH₃ |
| **(74)** | CF₂H | OH | NHCH₃ |
| **(75)** | OH | CF₂H | NHCH₃ |
| **(76)** | OCF₂H | OH | NHCH₃ |
| **(77)** | OH | OCF₂H | NHCH₃ |
| **(78)** | OH | OH | NHCH₃ |
| **(79)** | NH₂ | OH | NHCH₃ |
| **(80)** | OH | NHCH₃ | NHCH₃ |
| **(81)** | NHCH₃ | OH | NHCH₃ |
| **(82)** | CF₂H | OH | NHCH₃ |
| **(83)** | OH | CF₂H | NHCH₃ |
| **(84)** | CF₂H | CF₂H | NHCH₃ |
| **(85)** | OH | OCF₂H | NHCH₃ |
| **(86)** | OCF₂H | OH | NHCH₃ |
| **(87)** | OCF₂H | OCF₂H | NHCH₃ |
| **(88)** | OCF₂H | CF₂H | NHCH₃ |
| **(89)** | CF₂H | OCF₂H | NHCH₃ |

One aspect relates to a compound of formula (IIb): 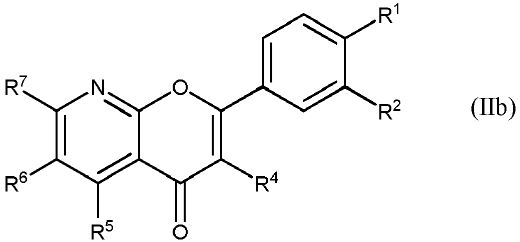 wherein
R¹, R² and R⁴ are independently selected from the group comprising hydroxyl, haloalkyl, haloalkoxy, heterocyclic, substituted heterocyclic, -NH₂, -NH(C₁-C₆ alkyl) and-N(C₁-C₆ alkyl)₂;
R⁵, R⁶ and R⁷ are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and-N(C₁-C₆ alkyl)₂.

One aspect relates to a compound of formula (IIc): 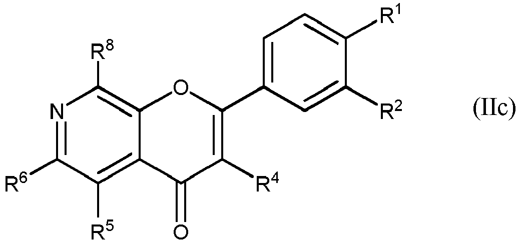 wherein
R¹, R² and R⁴ are independently selected from the group comprising hydroxyl, haloalkyl, haloalkoxy, -NH₂, heterocyclic, substituted heterocyclic, -NH(C₁-C₆ alkyl) and-N(C₁-C₆ alkyl)₂;
R⁵, R⁶ and R⁸ are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and-N(C₁-C₆ alkyl)₂.

**Table 3 - Non-limiting examples of compounds in accordance with formula (IIc) wherein each of R⁵, R⁶ and R⁸ are H.**

| | **R¹** | **R²** | **R⁴** |
|---|---|---|---|
| **(90)** | OH | NH₂ | OH |
| **(91)** | NH₂ | OH | OH |
| **(92)** | OH | NHCH₃ | OH |
| **(93)** | NHCH₃ | OH | OH |
| **(94)** | CF₂H | OH | OH |
| **(95)** | OH | CF₂H | OH |
| **(96)** | CF₂H | CF₂H | OH |
| **(97)** | OH | OCF₂H | OH |
| **(98)** | OCF₂H | OH | OH |
| **(99)** | OCF₂H | OCF₂H | OH |
| **(100)** | OCF₂H | CF₂H | OH |
| **(101)** | CF₂H | OCF₂H | OH |
| **(102)** | OH | NH₂ | OH |
| **(103)** | OH | OH | NH₂ |
| **(104)** | NH₂ | OH | NH₂ |
| **(105)** | OH | NHCH₃ | NH₂ |
| **(106)** | NHCH₃ | OH | NH₂ |
| **(107)** | CF₂H | OH | NH₂ |
| **(108)** | OH | CF₂H | NH₂ |
| **(109)** | CF₂H | CF₂H | NH₂ |
| **(110)** | OH | OCF₂H | NH₂ |
| **(111)** | OCF₂H | OH | NH₂ |
| **(112)** | OCF₂H | OCF₂H | NH₂ |
| **(113)** | OCF₂H | CF₂H | NH₂ |
| **(114)** | CF₂H | OCF₂H | NH₂ |
| **(115)** | OH | OH | NHCH₃ |
| **(116)** | OH | NH₂ | NHCH₃ |
| **(117)** | NH₂ | OH | NHCH₃ |
| **(118)** | CF₂H | OH | NHCH₃ |
| **(119)** | OH | CF₂H | NHCH₃ |
| **(120)** | OCF₂H | OH | NHCH₃ |
| **(121)** | OH | OCF₂H | NHCH₃ |
| **(122)** | OH | OH | NHCH₃ |
| **(123)** | NH₂ | OH | NHCH₃ |
| **(124)** | OH | NHCH₃ | NHCH₃ |
| **(125)** | NHCH₃ | OH | NHCH₃ |
| **(126)** | CF₂H | OH | NHCH₃ |
| **(127)** | OH | CF₂H | NHCH₃ |
| **(128)** | CF₂H | CF₂H | NHCH₃ |
| **(129)** | OH | OCF₂H | NHCH₃ |
| **(130)** | OCF₂H | OH | NHCH₃ |
| **(131)** | OCF₂H | OCF₂H | NHCH₃ |
| **(132)** | OCF₂H | CF₂H | NHCH₃ |
| **(133)** | CF₂H | OCF₂H | NHCH₃ |

One aspect relates to a compound of the formula (IId): 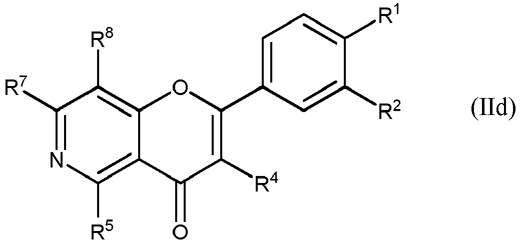 wherein
R¹, R² and R⁴ are independently selected from the group comprising hydroxyl, haloalkyl, haloalkoxy, -NH₂, heterocyclic, substituted heterocyclic, -NH(C₁-C₆ alkyl) and - N(C₁-C₆ alkyl)₂;
R⁵, R⁷ and R⁸ are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and -N(C₁-C₆ alkyl)₂.

**Table 4 - Non-limiting examples of compounds in accordance with formula (IId) wherein each of R⁵, R⁷ and R⁸ are H.**

| | **R¹** | **R²** | **R⁴** |
|---|---|---|---|
| **(134)** | OH | NH₂ | OH |
| **(135)** | NH₂ | OH | OH |
| **(136)** | OH | NHCH₃ | OH |
| **(137)** | NHCH₃ | OH | OH |
| **(138)** | CF₂H | OH | OH |
| **(139)** | OH | CF₂H | OH |
| **(140)** | CF₂H | CF₂H | OH |
| **(141)** | OH | OCF₂H | OH |
| **(142)** | OCF₂H | OH | OH |
| **(143)** | OCF₂H | OCF₂H | OH |
| **(144)** | OCF₂H | CF₂H | OH |
| **(145)** | CF₂H | OCF₂H | OH |
| **(146)** | OH | NH₂ | OH |
| **(147)** | OH | OH | NH₂ |
| **(148)** | NH₂ | OH | NH₂ |
| **(149)** | OH | NHCH₃ | NH₂ |
| **(150)** | NHCH₃ | OH | NH₂ |
| **(151)** | CF₂H | OH | NH₂ |
| **(152)** | OH | CF₂H | NH₂ |
| **(153)** | CF₂H | CF₂H | NH₂ |
| **(154)** | OH | OCF₂H | NH₂ |
| **(155)** | OCF₂H | OH | NH₂ |
| **(156)** | OCF₂H | OCF₂H | NH₂ |
| **(157)** | OCF₂H | CF₂H | NH₂ |
| **(158)** | CF₂H | OCF₂H | NH₂ |
| **(159)** | OH | OH | NHCH₃ |
| **(160)** | OH | NH₂ | NHCH₃ |
| **(161)** | NH₂ | OH | NHCH₃ |
| **(162)** | CF₂H | OH | NHCH₃ |
| **(163)** | OH | CF₂H | NHCH₃ |
| **(164)** | OCF₂H | OH | NHCH₃ |
| **(165)** | OH | OCF₂H | NHCH₃ |
| **(166)** | OH | OH | NHCH₃ |
| **(167)** | NH₂ | OH | NHCH₃ |
| **(168)** | OH | NHCH₃ | NHCH₃ |
| **(169)** | NHCH₃ | OH | NHCH₃ |
| **(170)** | CF₂H | OH | NHCH₃ |
| **(171)** | OH | CF₂H | NHCH₃ |
| **(172)** | CF₂H | CF₂H | NHCH₃ |
| **(173)** | OH | OCF₂H | NHCH₃ |
| **(174)** | OCF₂H | OH | NHCH₃ |
| **(175)** | OCF₂H | OCF₂H | NHCH₃ |
| **(176)** | OCF₂H | CF₂H | NHCH₃ |
| **(177)** | CF₂H | OCF₂H | NHCH₃ |

One aspect relates to a compound of the formula (IIe): 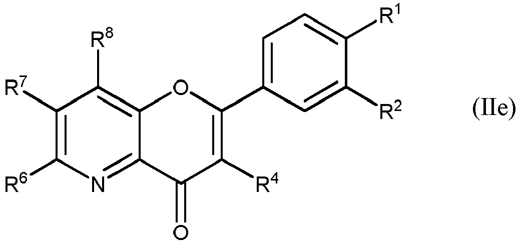 wherein
R¹, R² and R⁴ are independently selected from the group comprising hydroxyl, haloalkyl, haloalkoxy, -NH₂, heterocyclic, substituted heterocyclic, -NH(C₁-C₆ alkyl) and-N(C₁-C₆ alkyl)₂;
R⁶, R⁷ and R⁸ are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and -N(C₁-C₆ alkyl)₂.

One aspect relates to a compound of the formula (IIf): 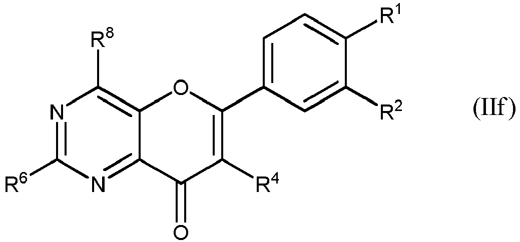 wherein
R¹, R² and R⁴ are independently selected from the group comprising hydroxyl, haloalkyl, haloalkoxy, -NH₂, heterocyclic, substituted heterocyclic, -NH(C₁-C₆ alkyl) and-N(C₁-C₆ alkyl)₂;
R⁶ and R⁸ are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and-N(C₁-C₆ alkyl)₂.

One aspect relates to a compound of the formula (IIg): 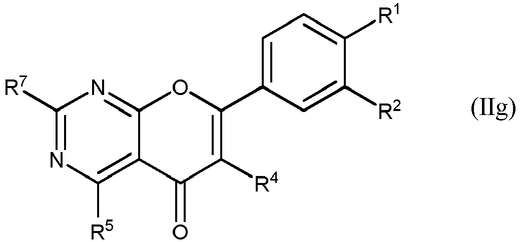 wherein
R¹, R² and R⁴ are independently selected from the group comprising hydroxyl, haloalkyl, haloalkoxy, -NH₂, heterocyclic, substituted heterocyclic, -NH(C₁-C₆ alkyl) and-N(C₁-C₆ alkyl)₂;
R⁵ and R⁷ are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and-N(C₁-C₆ alkyl)₂.

One aspect relates to a compound of formula (III): 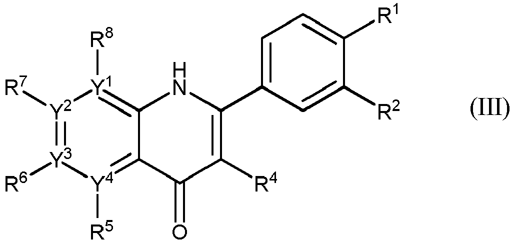 wherein
Y¹, Y², Y³, Y⁴ are independently selected from C or N;
R¹, R² and R⁴ are independently selected from the group comprising hydroxyl, haloalkyl, haloalkoxy, heterocyclic, substituted heterocyclic, -NH₂, -NH(C₁-C₆ alkyl) and-N(C₁-C₆ alkyl)₂;
R⁵, R⁶, R⁷ and R⁸ where present are H,; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and-N(C₁-C₆ alkyl)₂.

One aspect relates to a compound of formula (IIIa): 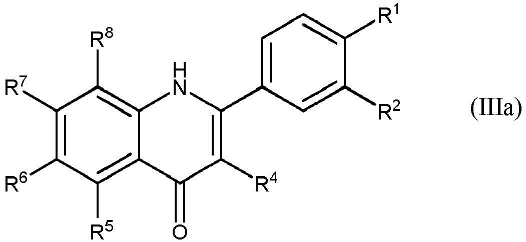 wherein
R¹, R² and R⁴ are independently selected from the group comprising hydroxyl, haloalkyl, haloalkoxy, -NH₂, heterocyclic, substituted heterocyclic, -NH(C₁-C₆ alkyl) and-N(C₁-C₆ alkyl)₂;
R⁵, R⁶, R⁷ and R⁸ are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and -N(C₁-C₆ alkyl)₂.

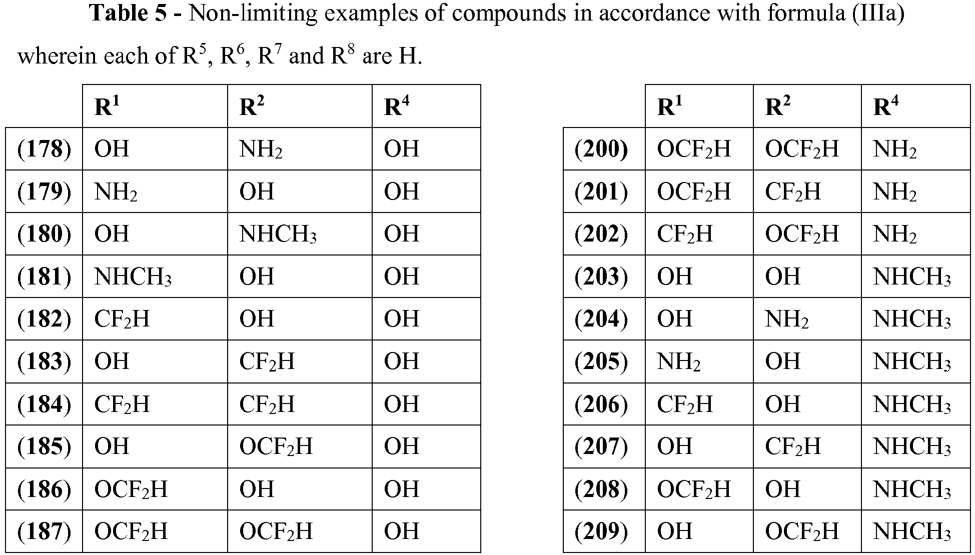

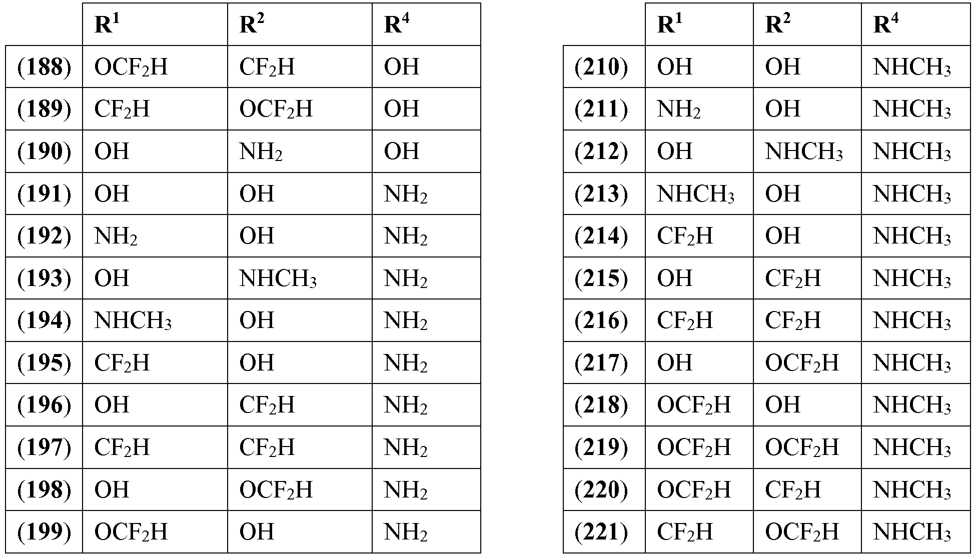

One aspect relates to a compound of formula (IIIb): 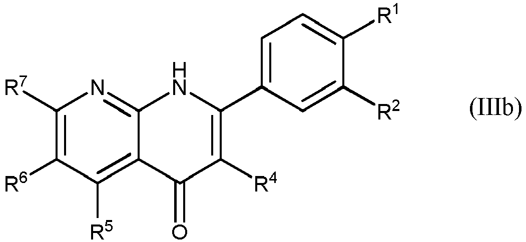 wherein
R¹, R² and R⁴ are independently selected from the group comprising hydroxyl, haloalkyl, haloalkoxy, heterocyclic, substituted heterocyclic, -NH₂, -NH(C₁-C₆ alkyl) and-N(C₁-C₆ alkyl)₂;
R⁵, R⁶ and R⁷ are H; or
a pharmaceutically acceptable salt, hydrate and/or solvate thereof;
with the *proviso* that at least one of R¹, R² and/or R⁴ is selected from the group comprising haloalkyl, haloalkoxy, heterocyclic, substituted heterocyclic, -NH₂, -NH(C₁-C₆ alkyl) and -N(C₁-C₆ alkyl)₂.

One aspect relates to a compound of formula (IIIc): 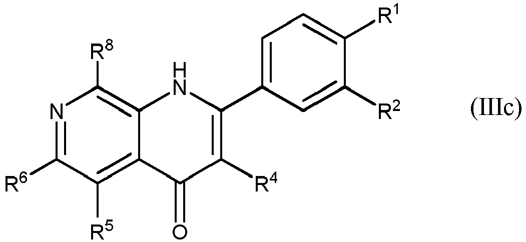 wherein
R¹, R² and R⁴ are independently selected from the group comprising hydroxyl, haloalkyl, haloalkoxy, heterocyclic, substituted heterocyclic, -NH₂, -NH(C₁-C₆ alkyl) and-N(C₁-C₆ alkyl)₂;
R⁵, R⁶ and R⁸ are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and -N(C₁-C₆ alkyl)₂.

One aspect relates to a compound of the formula (IIId): 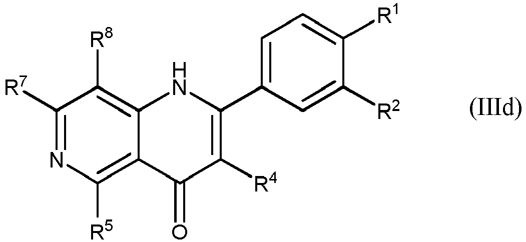 wherein
R¹, R² and R⁴ are independently selected from the group comprising hydroxyl, haloalkyl, haloalkoxy, heterocyclic, substituted heterocyclic, -NH₂, -NH(C₁-C₆ alkyl) and-N(C₁-C₆ alkyl)₂;
R⁵, R⁷ and R⁸ are H,; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and -N(C₁-C₆ alkyl)₂.

One aspect relates to a compound of the formula (IIIe): 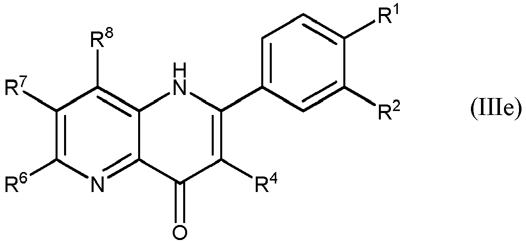 wherein
R¹, R² and R⁴ are independently selected from the group comprising hydroxyl, haloalkyl, haloalkoxy, heterocyclic, substituted heterocyclic, -NH₂, -NH(C₁-C₆ alkyl) and-N(C₁-C₆ alkyl)₂;
R⁶, R⁷ and R⁸ are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and -N(C₁-C₆ alkyl)₂.

One aspect relates to a compound of the formula (IIIf): 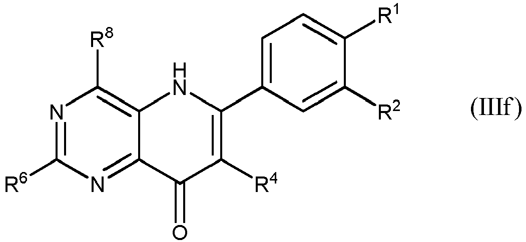 wherein
R¹, R² and R⁴ are independently selected from the group comprising hydroxyl, haloalkyl, haloalkoxy, heterocyclic, substituted heterocyclic, -NH₂, -NH(C₁-C₆ alkyl) and - N(C₁-C₆ alkyl)₂;
R⁶ and R⁸ are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and-N(C₁-C₆ alkyl)₂.

One aspect relates to a compound of the formula (IIIg): 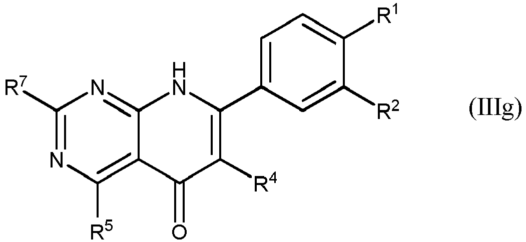 wherein
R¹, R² and R⁴ are independently selected from the group comprising hydroxyl, haloalkyl, haloalkoxy, heterocyclic, substituted heterocyclic, -NH₂, -NH(C₁-C₆ alkyl) and-N(C₁-C₆ alkyl)₂;
R⁵ and R⁷ are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and -N(C₁-C₆ alkyl)₂.

One aspect relates to a compound of formula (IV): 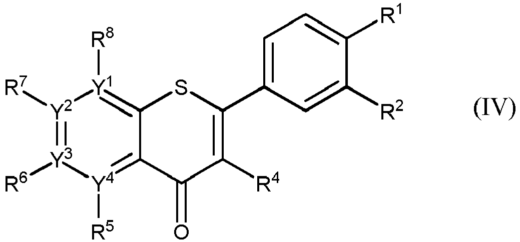 wherein
Y¹, Y², Y³, Y⁴ are independently selected from C or N;
R¹, R² and R⁴ are independently selected from the group comprising hydroxyl, haloalkyl, haloalkoxy, heterocyclic, substituted heterocyclic, -NH₂, -NH(C₁-C₆ alkyl) and-N(C₁-C₆ alkyl)₂;
R⁵, R⁶, R⁷ and R⁸ where present are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and -N(C₁-C₆ alkyl)₂.

One aspect relates to a compound of formula (IVa): 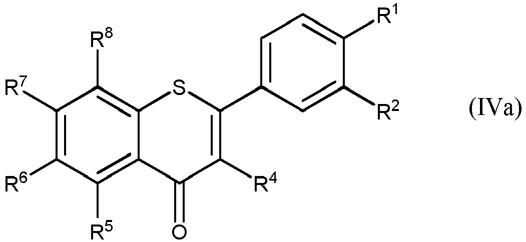 wherein
R¹, R² and R⁴ are independently selected from the group comprising hydroxyl, haloalkyl, haloalkoxy, heterocyclic, substituted heterocyclic, -NH₂, -NH(C₁-C₆ alkyl) and-N(C₁-C₆ alkyl)₂;
R⁵, R⁶, R⁷ and R⁸ are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and -N(C₁-C₆ alkyl)₂.

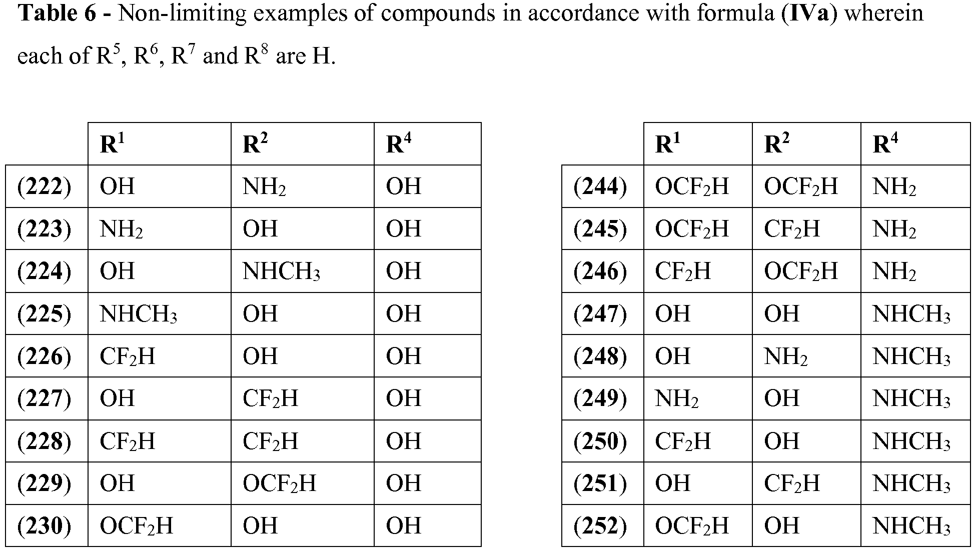

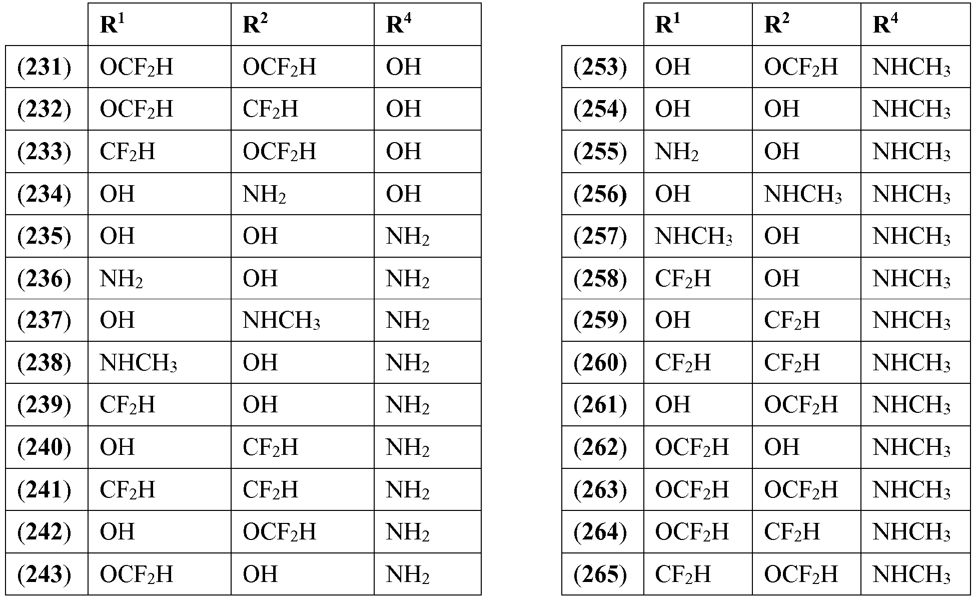

One aspect relates to a compound of formula (IVb): 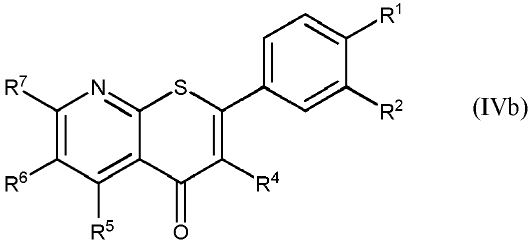 wherein
R¹, R² and R⁴ are independently selected from the group comprising hydroxyl, haloalkyl, haloalkoxy, heterocyclic, substituted heterocyclic, -NH₂, -NH(C₁-C₆ alkyl) and-N(C₁-C₆ alkyl)₂;
R⁵, R⁶ and R⁷ are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and -N(C₁-C₆ alkyl)₂.

One aspect relates to a compound of formula (IVc): 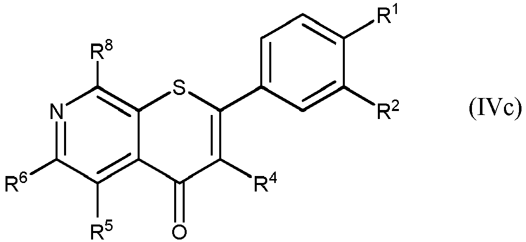 wherein
R¹, R² and R⁴ are independently selected from the group comprising hydroxyl, haloalkyl, haloalkoxy, heterocyclic, substituted heterocyclic, -NH₂, -NH(C₁-C₆ alkyl) and-N(C₁-C₆ alkyl)₂;
R⁵, R⁶ and R⁸ are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and -N(C₁-C₆ alkyl)₂.

One aspect relates to a compound of the formula (IVd): 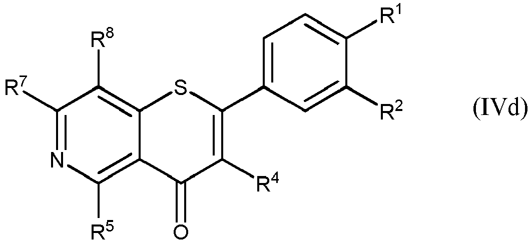 wherein
R¹, R² and R⁴ are independently selected from the group comprising hydroxyl, haloalkyl, haloalkoxy, heterocyclic, substituted heterocyclic, -NH₂, -NH(C₁-C₆alkyl) and-N(C₁-C₆ alkyl)₂;
R⁵, R⁷ and R⁸ are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and -N(C₁-C₆ alkyl)₂.

One aspect relates to a compound of the formula (IVe): 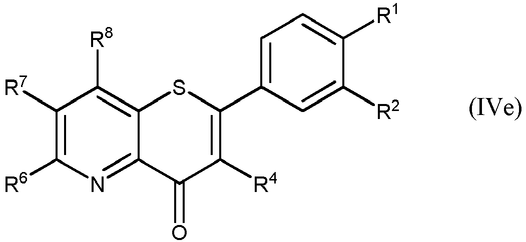 wherein
R¹, R² and R⁴ are independently selected from the group comprising hydroxyl, haloalkyl, haloalkoxy, heterocyclic, substituted heterocyclic, -NH₂, -NH(C₁-C₆ alkyl) and-N(C₁-C₆ alkyl)₂;
R⁶, R⁷ and R⁸ are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and -N(C₁-C₆ alkyl)₂.

One aspect relates to a compound of the formula (IVf): 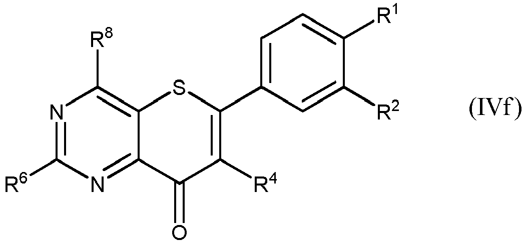 wherein
R¹, R² and R⁴ are independently selected from the group comprising hydroxyl, haloalkyl, haloalkoxy, heterocyclic, substituted heterocyclic, -NH₂, -NH(C₁-C₆ alkyl) and - N(C₁-C₆ alkyl)₂;
R⁶ and R⁸ are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆alkyl) and -N(C₁-C₆ alkyl)₂.

One aspect relates to a compound of the formula (IVg): 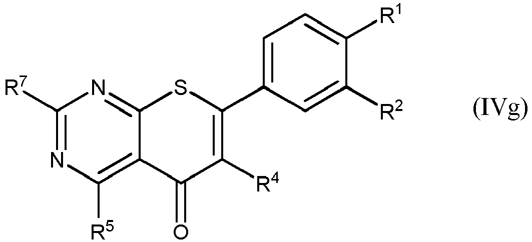 wherein
R¹, R² and R⁴ are independently selected from the group comprising hydroxyl, haloalkyl, haloalkoxy, heterocyclic, substituted heterocyclic, -NH₂, -NH(C₁-C₆ alkyl) and-N(C₁-C₆ alkyl)₂;
R⁵ and R⁷ are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and -N(C₁-C₆ alkyl)₂.

One aspect relates to a compound in accordance with formula (**I**), (**II**), (**IIa**) to (**IIg**), (**III**), (**IIIa**) to (**IIIg**), (**IV**), (**IVa**) to (**IVg**) for use in the prevention and/or mitigation of a disease caused by cell apoptosis and/or cell necrosis;
wherein, where present, X, Y¹, Y², Y³, Y⁴, R¹, R², R³, R⁴, R⁵, R⁶, R⁷ and R⁸ are defined as above;
or a pharmaceutically acceptable salt, hydrate and/or solvate thereof.

A method of inhibiting pro-apoptotic signalling kinases p38α and JNK is also disclosed, the method comprising:
administering any one or more of the compound(s) in accordance with formula (**I**), (**II**), (**IIa**) to (**IIg**), (**III**), (**IIIa**) to (**IIIg**), (**IV**), (**IVa**) to (**IVg**);
wherein, where present, X, Y¹, Y², Y³, Y⁴, R¹, R², R³, R⁴, R⁵, R⁶, R⁷ and R⁸ are defined as above;
or a pharmaceutically acceptable salt, hydrate, solvate, prodrug and isomer thereof.

A method of activating extracellular-signal-regulated kinase (ERK) activity is also disclosed, the method comprising:
administering any one or more of the compound(s) in accordance with formula (I), (**II**), (**IIa**) to (**IIg**), (**III**), (**IIIa**) to (**IIIg**), (**IV**), (**IVa**) to (**IVg**);
wherein, where present, X, Y¹, Y², Y³, Y⁴, R¹, R², R³, R⁴, R⁵, R⁶, R⁷ and R⁸ are defined as above;
or a pharmaceutically acceptable salt, hydrate, solvate, prodrug and isomer thereof.

Without wishing to be bound by theory, it is thought that compounds of the present invention may act as both a p38α and JNK inhibitor and an activator of ERK activity.

A method of inhibiting p38α and JNK activity while simultaneously activating ERK activity is also disclosed, the method comprising
administering any one or more of the compound(s) in accordance with formula (**I**), (**II**), (**IIa**) to (**IIg**), (**III**), (**IIIa**) to (**IIIg**), (**IV**), (**IVa**) to (**IVg**);
wherein, where present, X, Y¹, Y², Y³, Y⁴, R¹, R², R³, R⁴, R⁵, R⁶, R⁷ and R⁸ are defined as above;
or a pharmaceutically acceptable salt, hydrate, solvate, prodrug and isomer thereof.

One aspect relates to a compound in accordance with formula (**I**), (**II**), (**IIa**) to (**IIg**), (**III**), (**IIIa**) to (**IIIg**), (**IV**), (**IVa**) to (**IVg**) for use in preventing and/or treating a disease in a subject associated with the presence of reactive oxidative species (ROS);
wherein, where present, X, Y¹, Y², Y³, Y⁴, R¹, R², R³, R⁴, R⁵, R⁶, R⁷ and R⁸ are defined as above;
or a pharmaceutically acceptable salt, hydrate and/or solvate thereof.

In one particular embodiment, the compound is selected from the group comprising:
3',4'-dihydroxy flavonol, 4'-hydroxy flavonol; and/ or
at least one compound (**1**) to (**281**), excluding compounds (46) to (89);
or a pharmaceutically acceptable salt, hydrate and/or solvate thereof.

In one embodiment, the subject in need of such treatment is at risk of developing ischaemia. In a particular embodiment, the subject is suffering ischaemia and/or reperfusion injury as a result of an acute or chronic condition.

A use of a compound for the manufacture of a medicament for the prevention and / or mitigation of damage caused by cell apoptosis or cell necrosis in a subject is also disclosed, wherein the compound is in accordance with formula (**I**), (**II**), (**IIa**) to (**IIg**), (**III**), (**IIIa**) to (**IIIg**), (**IV**), (**IVa**) to (**IVg**);
wherein, where present, X, Y¹, Y², Y³, Y⁴, R¹, R², R³, R⁴, R⁵, R⁶, R⁷ and R⁸ are defined as above;
or a pharmaceutically acceptable salt, hydrate, solvate, prodrug and isomer thereof.

A use of a compound for the manufacture of a medicament for inhibiting pro-apoptotic signalling kinases p38α and JNK activity in a subject is also disclosed, wherein the compound is in accordance with formula (**I**), (**II**), (**IIa**) to (**IIg**), (**III**), (**IIIa**) to (**IIIg**), (**IV**), (**IVa**) to (**IVg**);
wherein, where present, X, Y¹, Y², Y³, Y⁴, R¹, R², R³, R⁴, R⁵, R⁶, R⁷ and R⁸ are defined as above;
or a pharmaceutically acceptable salt, hydrate, solvate, prodrug and isomer thereof.

A use of a compound for the manufacture of a medicament for activating extracellular-signal-regulated kinase (ERK) activity in a subject is also disclosed, wherein the compound is in accordance with formula (**I**), (**II**), (**IIa**) to (**IIg**), (**III**), (**IIIa**) to (**IIIg**), (**IV**), (**IVa**) to (**IVg**);
wherein, where present, X, Y¹, Y², Y³, Y⁴, R¹, R², R³, R⁴, R⁵, R⁶, R⁷ and R⁸ are defined as above;
or a pharmaceutically acceptable salt, hydrate, solvate, prodrug and isomer thereof.

A use of a compound for the manufacture of a medicament for inhibiting p38α and JNK activity while simultaneously activating ERK activity in a subject is also disclosed, wherein the compound is in accordance with formula (**I**), (**II**), (**IIa**) to (**IIg**), (**III**), (**IIIa**) to (**IIIg**), (**IV**), (**IVa**) to (**IVg**);
wherein, where present, X, Y¹, Y², Y³, Y⁴, R¹, R², R³, R⁴, R⁵, R⁶, R⁷ and R⁸ are defined as above;
or a pharmaceutically acceptable salt, hydrate, solvate, prodrug and isomer thereof.

A use of a compound for the manufacture of a medicament for the treatment and/or of a disease(s) in a subject associated with the presence of reactive oxidative species (ROS) is also disclosed, wherein the compound is in accordance with formula (**I**), (**II**), (**IIa**) to (**IIg**), (**III**), (**IIIa**) to (**IIIg**), (**IV**), (**IVa**) to (**IVg**);
wherein, where present, X, Y¹, Y², Y³, Y⁴, R¹, R², R³, R⁴, R⁵, R⁶, R⁷ and R⁸ are defined as above;
or a pharmaceutically acceptable salt, hydrate, solvate, prodrug and isomer thereof.

Broadly, the compounds and compositions of the present invention can be used to treat or prevent cell damage or death due to necrosis or apoptosis, cerebral ischemia and reperfusion injury or neurodegenerative diseases in an animal, such as a human. The compounds and compositions of the present invention can be used to extend the lifespan and proliferative capacity of cells and thus can be used to treat or prevent diseases associated therewith; they alter gene expression of senescent cells; and they radiosensitize hypoxic tumor cells. Preferably, the compounds and compositions of the invention can be used to treat or prevent tissue damage resulting from cell damage or death due to necrosis or apoptosis, and/or effect neuronal activity.

While not wishing to be bound by theory, it is thought that the compounds of the present invention can also assist in maintaining and/or improving circulatory flow. For example, the compounds of the present invention may be administered to a patient with diabetes to assist in management of the disease.

The chronic condition may be selected from cancer, cerebrovascular disease, pulmonary vascular disease, atherosclerosis, artery disease, congestive heart disease, coronary disease, peripheral vascular disease, diabetes, hypertension, migraine, burns, chronic obstructive pulmonary disease and retinal vascular disease.

The acute condition may be selected from stroke, myocardial infarction, mechanical trauma resulting from crush injury or surgery. In a particular embodiment, the vascular surgery is heart bypass and/or transplant surgery.

The compound may be administered to the subject before and/or during the surgery.

A method of preventing, delaying the onset of and/or slowing the progression of atherosclerosis and/or coronary heart disease in a subject is also disclosed, the method comprising
administering any one or more of the compound(s) in accordance with formula **(I), (II), IIa)** to **(IIg), (III), (IIIa)** to **(IIIg), (IV), (IVa)** to **(IVg);**
wherein, where present, X, Y¹, Y², Y³, Y⁴, R¹, R², R³, R⁴, R⁵, R⁶, R⁷ and R⁸ are defined as above;
or a pharmaceutically acceptable salt, hydrate, solvate, prodrug and isomer thereof

A therapeutic and/or prophylactic method of preventing and/or treating a disease(s) in a subject associated with the presence of reactive oxidative species (ROS) is also disclosed, the method comprising:
administering any one or more of the compound(s) in accordance with formula (**I**), **(II), (IIa)** to **(IIg), (III), (IIIa)** to **(IIIg), (IV), (IVa)** to **(IVg);**
wherein, where present, X, Y¹, Y², Y³, Y⁴, R¹, R², R³, R⁴, R⁵, R⁶, R⁷ and R⁸ are defined as above;
or a pharmaceutically acceptable salt, hydrate, solvate, prodrug and isomer thereof.

One aspect relates to a compound of formula **(I), (II), (Ha)** to **(IIg), (HI), (IIIa)** to **(IIIg), (IV), (IVa)** to **(IVg)** for use in preventing and/or mitigating ischaemia and/or reperfusion injury,
wherein, where present, X, Y¹, Y², Y³, Y⁴, R¹, R², R³, R⁴, R⁵, R⁶, R⁷ and R⁸ are defined as above;
or a pharmaceutically acceptable salt, hydrate and/or solvate thereof

One aspect relates to a compound of formula **(I), (II), (IIa)** to **(IIg), (III), (IIIa)** to **(IIIg), (IV), (IVa)** to **(IVg)** for use in ameliorating damage to a subject caused by the administration of a therapeutic agent, wherein, where present, X, Y¹, Y², Y³, Y⁴, R¹, R², R³, R⁴, R⁵, R⁶, R⁷ and R⁸ are defined as above; or a pharmaceutically acceptable salt, hydrate and/or solvate thereof

Therapeutic agent may be an oxidative therapeutic agent. A particular example of a therapeutic agent is an anticancer agent. In particular, the anticancer agent may be anthracycline and its derivatives.

In particular embodiments, the compound is administered orally, topically, subcutaneous, parenterally, intramuscular, intra-arterial and/or intravenously. In a particular embodiment, the compound is administered orally.

Also disclosed is a use of a compound as specified above for the preparation of a medicament.

Also disclosed is a method for synthesizing compounds as specified above.

The formulae given herein are intended to extend to all possible geometric and optical isomers as well as racemic mixtures thereof.

One aspect also relates to a pharmaceutical and/or a veterinary composition comprising a pharmaceutically and/or veterinarily acceptable carrier or diluent together with at least one compound in accordance with the present invention, or a pharmaceutically acceptable salt, hydrate and/or solvate thereof.

A method of preventing and/or at least ameliorating damage to a subject caused by the administration of a therapeutic agent is also disclosed, the method comprising coadministering to a subject:
i) a therapeutic agent; and
ii) a therapeutically effective amount of at least one compound in accordance with the present invention, or a pharmaceutically acceptable salt or solvates thereof.

A method of preventing and/or treating a disease(s) associated with the presence of reactive oxidative species (ROS) is disclosed, the method comprising administering a therapeutically effective amount of at least one compound in accordance with the present invention, or a pharmaceutically acceptable salt or solvates thereof.

Typically the subject in need of such treatment will be a person at risk of developing ischaemia. Alternatively, the subject may be a person who is currently suffering ischaemia and/or reperfusion as a result of an acute or chronic condition.

A method is disclosed of preventing and/or at least ameliorating the damage to a subject caused by ischaemia and/or reperfusion, the method comprising administering a therapeutically effective amount of at least one compound in accordance with the present invention as specified above, or a pharmaceutically acceptable salt or solvates thereof.

It is desirable that the presence of at least one solubilising group renders the compound at least partially soluble, and more preferably, totally soluble in aqueous solution, preferably water.

A method of treating a subject having a disease or disorder involving oxidative damage is also disclosed, the method comprising administering a therapeutically effective amount of the composition of the invention.

Preferably, the disease or disorder involving oxidative damage is selected from the group consisting of cancer, heart disease, neurological disorders, auto-immune disorders, ischaemia-reperfusion injury, diabetic complications, septic shock, hepatitis, atherosclerosis, Alzheimer's disease and complications arising from HIV or Hepatitis, including Hepatitis B.

In a particular embodiment, the subject is an animal. The animal may be selected from the group consisting of humans, non-human primates, cattle, horses, pigs, sheep, goats, dogs, cats, birds, chickens or other poultry, ducks, geese, pheasants, turkeys, quails, guinea pigs, rabbits, hamsters, rats and mice.

In some aspects, the one or more compounds of the present invention may be administered simultaneously, separately or sequentially with the one or more therapeutic agent(s).

When used in such a combination the one or more therapeutic agent(s) and the one or more compounds of the present invention may be administered as separate agents at the same or different times or they can be formulated as a single composition comprising both compounds.

Examples of relevant acute disorders causing the production of ROS include ischaemia reperfusion, stroke, myocardial infarction or mechanical trauma, such as a crush injury or surgery. Some forms of surgery such as heart bypass or transplant surgery necessarily cause ischaemia and reperfusion of tissue. Typically one or more flavonoid derivatives according to the present invention are administered to the subject before and/or during surgery.

Chronic disorders may be chosen from the group including cancer, cerebrovascular disease, atherosclerosis, artery disease including coronary disease, peripheral vascular disease (including damage caused by diseases such as diabetes), hypertension, pulmonary hypertension, chronic obstructive airways disease, emphysema, neurological disorders, auto-immune disorders, diabetic complications, septic and hypovolemic shock, burns, hepatitis, and complications arising from hepatitis and HIV. Another chronic disorder may be chosen from the complications resulting from administration of hyperbaric or high oxygen tension atmospheres, often applied to assist breathing particularly in a premature infant human, including retinal or other eye damage. Subjects at risk of relevant chronic disorders may be diagnosed by analysis of symptoms, diagnostic testing, enzymatic markers, or by genetic testing to identify a genetic predisposition. Predisposition to certain acute disorders such as heart attack or stroke may also be identified by genetic testing and may prompt the prophylactic application of one or more flavonoid derivatives to the subject at risk. Drug-induced disorders due to ROS e.g. drug induced congestive heart disease.

If the disease or disorder is stroke or risk or stroke, the composition described above is preferably administered before the stroke occurs as a prophylactic to reduce the risk of stroke occurrence, or within twelve hours (preferably within four hours) of stroke occurrence.

An example of an ROS involved pathological condition is ischaemia where a deficiency of blood flow to part of a body results in inadequate tissue perfusion with oxygen. Ischaemia causes tissue damage, the severity of the damage depending on the length of time the tissue is deprived of oxygen and whether adequate reperfusion of oxygen occurs after the ischaemic event.

At least one compound in accordance with the present invention may be administered via a number of different routes, for example, topically, orally, subcutaneous, intramuscular, intra-arterially and/or intravenously.

### Definitions

As used herein, the term "alkyl" includes branched or unbranched hydrocarbon chains, such as, methyl, ethyl, *n*-propyl, *iso*-propyl, *n*-butyl, sec-butyl, iso-butyl, tertbutyl, octa-decyl and 2-methylpentyl. These groups can be substituted or unsubstituted with one or more functional groups which are attached commonly to such chains, such as, hydroxyl, bromo, fluoro, chloro, iodo, mercapto or thio, cyano, alkylthio, heterocyclyl, aryl, heteroaryl, carboxyl, carbalkoyl, alkyl, alkenyl, nitro, amino, alkoxyl, amido, and the like to form alkyl groups such as trifluoromethyl, 3-hydroxyhexyl, 2-carboxypropyl, 2-fluoroethyl, carboxymethyl, cyanobutyl and the like.

The term "lower" herein includes a linear or branched chain of C₁ to C₆ carbon atoms.

The term "alkoxy" includes -OR-, wherein R is C₁-C₆ alkyl. The term "lower alkoxy radicals" there may be mentioned linear and branched alkoxy groups of 1 to 6 carbon atoms, such as methoxy, ethoxy, propoxy, isopropoxy, butoxy, isobutoxy, sec-butoxy, tert-butoxy, pentyloxy, isopentyloxy, hexyloxy and isohexyloxy groups.

Haloalkyl is alkyl substituted wholly or partly by halogen, preferably by fluorine, chlorine and/or bromine, in particular by fluorine, e.g. CF₃, CHF₂, CH₂F, CF₃CF₂, CH₂FCHCl, CCl₃, CHCl₂, and CH₂CH₂Cl.

The term "haloalkyl" as used herein refers to an alkyl group, as defined above, wherein one or more of the alkyl group's hydrogen atoms are independently replaced with a halogen. In one embodiment, a haloalkyl group is a "fluoroalkyl group."

The term "fluoroalkyl group" as used herein, refers to an alkyl group, wherein one or more of the alkyl group's hydrogen atoms are replaced with a --F atom. In various embodiments, a fluorooalkyl group contains one F atom, two F atoms or three F atoms. Illustrative example of fluoroalkyl groups include, but are not limited to, CH₂F, CHF₂, CH₂CH₂F, CF₃, and -(CH₂)₃CF₃.

The term "haloalkoxy" is used herein refers alkoxy group, as defined above, wherein one or more of the alkyl group's hydrogen atoms is independently replaced with a halogen. Illustrative examples of haloalkoxy include, but are not limited to, OCF₃, OCHF₂, OCH₂F, CF₃CF₂O, OCH₂CF₃ and OCH₂CH₂Cl; similar comments apply to other halogen-substituted radicals.

The term "heterocyclic" refers to a saturated or unsaturated group having a single ring or multiple condensed rings, from 1 to 10 ring carbon atoms and from 1 to 4 ring hetero atoms selected from nitrogen, sulfur or oxygen within the ring wherein, in fused ring systems, one or more of the rings can be aryl or heteroaryl. Examples of heterocycles and heteroaryls include, but are not limited to, azetidine, pyrrole, imidazole, pyrazole, pyridine, pyrazine, pyrimidine, pyridazine, indolizine, isoindole, indole, dihydroindole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, piperidine, piperazine, indoline, phthalimide, 1,2,3,4-tetrahydroisoquinoline, 4,5,6,7-tetrahydrobenzo[b]thiophene, thiazole, thiazolidine, thiophene, benzo[b]thiophene, morpholino, thiomorpholino, piperidinyl, pyrrolidine, tetrahydrofuranyl, and the like.

The term "substituted heterocyclic" refers to heterocycle groups, as defined above, which are substituted with from 1 to 3 substituents independently selected from the group consisting of oxo (=O), thioxo (=S), plus the same group of substituents as defined for substituted aryl.

Pharmaceutically acceptable esters of the present compounds may include the following groups: (1) carboxylic acid esters obtained by esterification of the hydroxy groups, in which the non-carbonyl moiety of the carboxylic acid portion of the ester grouping is selected from straight or branched chain alkyl (for example, acetyl, n-propyl, t-butyl, or n-butyl), alkoxyalkyl (for example, methoxymethyl), aralkyl (for example, benzyl), aryloxyalkyl (for example, phenoxymethyl), aryl (for example, phenyl optionally substituted with, for example, halogen, C₁₋₄alkyl, or C₁₋₄alkoxy or amino); (2) sulfonate esters, such as alkyl- or aralkylsulfonyl (for example, methanesulfonyl); (3) amino acid esters (for example, L-valyl or L-isoleucyl); (4) phosphonate esters and (5) mono-, di- or triphosphate esters. The phosphate esters may be further esterified by, for example, a C₁₋₂₀ alcohol or reactive derivative thereof, or by a 2,3-di(C₆₋₂₄)acyl glycerol.

### Sheep model of myocardial ischemia-reperfusion (I/R) injury

Studies were performed on adult merino ewes (35-45kg), that were randomly assigned to a treatment protocol. In anesthetised ewes, surgical ligation of the second diagonal branch of the left anterior descending coronary artery (D2) was performed. Ischemia (1 h) was induced by occluding the D2 with a vascular snare, and reperfusion was achieved by releasing the snare. Arrhythmias were managed by administration of lignocaine into the left atrium. The test compound (6.6 mg/kg) or vehicle (10 ml 0.1 M Na₂CO₃) were administered 5 minutes prior to reperfusion in the different treatment groups (10, 30, 60 or 180 min). Sheep were killed with an intravenous overdose of pentobarbitone (100 mg/kg) at the end of the ischemic period and after 10, 30, 60 or 180 min of reperfusion.

Myocardial area-at-risk (AAR) was determined by Evan's blue staining, with the infarct tissue delineated by triphenyltetrazolium chloride (TTC). After reperfusion, the D2 was re-occluded before Evan's blue dye (1.5%, 40 ml) was injected simultaneously with pentobarbitone (100 mg/kg). The heart was excised, 1 cm transverse sections were made of the left ventricle and AAR regions were recorded by photography before immersion of the sections in TTC. Viable tissue was stained with TTC, and these regions were similarly photographed. Computerised planimetry (MCID-M2) was used to measure the area of infarct relative to AAR. Tissue from the non-infarcted AAR was dissected from the myocardial rings. Protein extraction for immunoblot analysis was conducted as follows, AAR samples were snap frozen in liquid nitrogen, ground into a fine powder, and subsequently lysed with ice cold RIPA buffer (50 mM Tris-HCl pH 7.3, 150 mM NaCl, 0.1 mM EDTA, 1% [w/v] sodium deoxycholate, 1% (v/v) Triton X-100, 0.2% (w/v) NaF and 100 µM Na₃VO₄) in a 5:1 (w/v) ratio for subsequent immunoblot analysis.

### Cardiac myocyte isolation, cell culture and treatments

Cardiac myocytes were plated on collagen-coated dishes in serum containing media Dulbecco's modified Eagle's medium/Medium 199 (4:1 v/v) containing 10% (v/v) horse serum, 5% (v/v) fetal calf serum, and penicillin/streptomycin (100 units/ml). Murine skeletal myoblast (C2C12) or rat cardiac myoblasts (H9C2) were maintained in DMEM supplemented with 10% (v/v) fetal calf serum and 100 U/ml penicillin-streptomycin. Cells were incubated overnight at 37°C in a 5% CO₂ incubator prior to the experiment. Cells were pre-treated with flavonols for 30 min prior to stress treatments. Following pre-treatment, cells were stimulated by the addition of H₂O₂ (ImM), sorbitol (0.5 M) or arsenite (300 µM). DMSO (0.1% v/v) was used as a vehicle control. Following treatment, cells were assessed for viability or protein lysates prepared for immunoblot analysis.

### XTT cell viability assay

Cell proliferation was determined by labeling cells with the yellow tetrazolium salt, XTT (sodium 3'-[1-(phenylaminocarbonyl)-3,4-tetrazolium]-bis(4-methoxy-6-nitro) benzene sulfonic acid 5 hydrate), using the XTT Cell Proliferation Kit (Roche) according to manufacturer specifications. Briefly, 2x105 cells were seeded per well in a 96-well plate. Following cell treatments, the XTT labeling reagent was added during the final 2 h then the absorbance measured at 492 nm to indicate cell numbers and absorbance readings at 690 nm used as a reference (Abs₄₉₂ₙₘ - Abs₆₉₀ₙₘ). Background absorbance (XTT reagent incubated in absence of cells) was subtracted from all values and cell viability determined by dividing flavonol and stress-treated values by vehicle control.

### LDH cell survival assay

Cells (0.5x10⁵/well) were seeded per well on a 96-well plate. Following cell treatment, the cellular release of lactate dehydrogenase (LDH) was determined by measuring LDH activity in conditioned media using a cytotoxicity detection kit (LDH Release Assay, Roche Applied Science). In brief, at the end of the treatment period, supernatant (50 µl) was transferred to a new plate and a tetrazolium salt, INT (2-(4-Iodophenyl)-3-[(4-nitrophenyl)-5-phenyl]-2H-tetrazolium chloride) mixture (50 µl/well) added and incubated under low light for 30 min before measuring absorbance (490 nm, reference wavelength 650 nm). Maximum LDH release was achieved by the addition of 1% (w/v) Triton X-100. Percentage LDH release was determined from the ratio of sample_{Abs}/maximum possible LDH release_{Abs}.

### Cell lysate preparation and immunoblotting

Cell lysates were prepared in RIPA buffer (50 mM Tris-HCl pH 7.3, 150 mM NaCl, 0.1 mM EDTA, 1% [w/v] sodium deoxycholate, 1% (v/v) Triton X-100, 0.2% (w/v) NaF and 100 µM Na₃VO₄) supplemented with protease inhibitors. After 10 min on ice, cell debris was removed by centrifugation (14 000 x g, 10 min). Protein concentrations were then determined by Bradford assay. Protein lysates were resolved by SDS-PAGE transferred onto polyvinylidene fluoride membranes and immunoblotted [22]. Quantitation to determine relative band intensities was conducted using Image J (National Institutes of Health) and phospho-MAPK normalised against total protein.

### Cytoprotection against oxidative and chemical stress stimuli

To define the protective and kinase modulatory properties of the compounds of the certain embodiments, their effects in greater detail *in vitro.* The protection of cultured C2C12 murine myoblasts was evaluated against detrimental stress stimuli using an XTT assay of cell viability. In a time-course of oxidative damage, it was observed that 2 h treatment of H₂O₂ (0.5 or 1.0 mM) reduced myoblast viability to 38.5+2.0% and 31.9+0.8%, respectively, compared with control cells without H₂O₂ treatment. Pretreatment with certain compounds of the invention (10 µM) significantly improved the viability of C2C12 cells challenged with 0.5 or 1 mM H₂O₂. Assays of stress-stimulated cell membrane permeability and cell death as assessed by LDH activity assays of the media confirmed pretreatment significantly decreased LDH release in the presence of 0.5 or 1.0 mM H₂O₂. DiOHF was similarly protective in primary neonatal rat cardiac myocytes and H9C2 rat myoblasts.

The effect on cell death induced by arsenite, a chemical toxin that induces cell death through p38MAPK and JNK activation was then investigated. In time-course and dose-response studies, C2C12 viability was substantially reduced by arsenite treatment. Pretreatment with compounds of the invention (10 µM) significantly reduced arsenite-induced cell loss, with 66.9+5.2% of cells remaining viable compared with 27.0+3.9% viability when cells were pretreated with vehicle (DMSO). These data indicate that pretreatment was cytoprotective against both H₂O₂ and arsenite stress stimulation.

### JNK inhibition and p38α pathway activation by H₂O₂ and arsenite

It has been proposed that following I/R, decisions regarding cell survival or death are dependent on the relative activation of myocardial JNK, p38α, ERK and AKT. In particular, the activities of these kinases in the salvageable non-infarcted AAR are likely to be important determinants of the final infarct size. To assess whether the time course of activation of these kinases varies following reperfusion, we assessed their phosphorylation status as an indication of activation at 0, 10, 30 and 60 min of reperfusion. Increased kinase phosphorylation was observed from 10 min reperfusion and peaked at 30 min. For the stress-activated kinases, p38α and JNK, their phosphorylation at 30min was increased 5.8-fold and 6.2-fold, respectively, over ischemia only before decreasing by 1 h reperfusion. Similarly, elevated phosphorylation of ERK and AKT peaked at 11.7-fold and 11.9-fold by 30 min reperfusion, respectively, before trending towards basal levels at 1 h reperfusion. These findings therefore highlight the rapid activation of p38α, JNK, and ERK as well as AKT in the AAR region upon reperfusion.

To examine the impact of certain compounds described herein, treatment on SAPKs and AKT activation in response to I/R, we compared their activation at the time of peak phosphorylation (i.e. at 30 min reperfusion). Myocardial p38α and JNK phosphorylation levels in the non-infarcted AAR region were significantly lower in sheep pretreated than the equivalent vehicle-pretreated animals. Thus, our studies in this large animal model of acute myocardial I/R reveal that administration of certain compounds described herein, *in vivo,* inhibited p38α and JNK activation at 30 min reperfusion.

Activation of p38α and JNK by stress in murine myoblasts was inhibited, by pretreatment, in a similar manner to the effects noted during I/R in sheep. Both H₂O₂ and arsenite-stimulated increases in p38α and JNK phosphorylation in murine myoblasts were significantly inhibited by pretreatment. Similarly, we confirmed that pretreatment inhibited stress-stimulated p38α and JNK activation in primary cardiac myocytes. In contrast, pretreatment did not significantly reduce ERK phosphorylation stimulated by H₂O₂ or arsenite.

In summary, our findings indicate that compounds described herein prevented the progressive increase in infarct size in an ovine model of myocardial ischaemia and reperfusion. In the non-infarcted area at risk, the area where tissue can be salvaged, treatment reduced activation of p38α and JNK. Importantly, in cultured myoblasts and neonatal cardiac myocytes, pretreatment with compounds described herein also protected against oxidative and chemotoxic stress, and similarly decreased activation of p38α and JNK. Our cellular studies demonstrated detrimental effects of p38α and JNK activation in response to cellular stress, suggesting that activation of these SAPKs contributed to the development of I/R injury and cell loss *in vivo.* These findings highlight the potent cardioprotective action and demonstrate the therapeutic benefits of attenuating excessive stress-activated protein kinase (SAPK) activation in response to stress.

### Compositions and Methods

The compounds of this invention can be formulated in a variety of carriers and delivery systems. The amount of the therapeutic compound to be administered and the compound's concentration is dependent on the vehicle or device selected, the clinical condition of the patient, the side effects and the stability of the compound in the formulation. Thus, the physician employs the appropriate preparation containing the appropriate concentration of the therapeutic compound and selects the amount of formulation administered, depending upon clinical experience with the patient in question or with similar patients.

Furthermore, excipients can be included in the formulation. Examples include cosolvents, surfactants, oils, humectants, emollients, preservatives, stabilizers and antioxidants. Any pharmacologically acceptable buffer may be used, e.g., tris or phosphate buffers. Effective amounts of diluents, additives and excipients are those amounts which are effective to obtain a pharmaceutically acceptable formulation in terms of solubility, biological activity, etc.

Thus, a composition of the invention includes a therapeutic compound which can be formulated with conventional, pharmaceutically acceptable, vehicles for topical, oral or parenteral administration. Formulations can also include small amounts of adjuvants such as buffers and preservatives to maintain isotonicity, physiological and pH stability.

The compounds may be administered to both human and non-human subjects.

The compounds may be administered in compositions wherein the active compound is intimately admixed with one or more inert ingredients and optionally including one or more additional active ingredients. The compounds may be used in any composition known to those skilled in the art for administration to humans and animals.

The compositions may be administered through a proper route according to the dosage form. For example, the injection can be administered intravenous, intra-arterial, subcutaneous, intramuscular and the like.

For oral administration, either solid or fluid unit dosage forms can be prepared. The water soluble forms can be dissolved in an aqueous vehicle together with sugar, aromatic flavouring agents and preservatives to form syrup. An elixir is prepared by using a hydroalcoholic (e.g., ethanol) vehicle with suitable sweeteners such as sugar and saccharin, together with an aromatic flavouring agent. Suspensions can be prepared with an aqueous vehicle with the aid of a suspending agent such as acacia, tragacanth, methylcellulose and the like. The synthetic flavonoid compounds of the present invention may also be formulated with stabilizing agents, for example metal chelator reducing agents such as ethylenediaminetetracetic acid (EDTA) or a reducing agent such as sodium metabisufite.

Appropriate formulations for parenteral use are apparent to the practitioner of ordinary skill. Usually, the therapeutic compound is prepared in an aqueous solution in a concentration of from about 1 to about 100 mg/mL. More typically, the concentration is from about 10 to 60 mg/mL or about 20 mg/mL. Concentrations below 1 mg/mL may be necessary in some cases depending on the solubility and potency of the compound selected for use. The formulation, which is sterile, is suitable for various parenteral routes including intra-dermal, intra-articular, intramuscular, intravascular, intravenous, inhalation and subcutaneous.

Compositions may be formulated into sunscreens, skin care compositions, emollient of moisturizers.

The synthetic flavonoid compound(s) may also be formulated as a nutrapharmaceutical or a nutraceutical. For example, the synthetic flavonoid compound(s) may be formulated into a food, such as a cereal, beverages such as fruit juice, alcoholic drinks, bread, etc, for oral consumption.

### Non-limiting examples

The following examples further illustrate the present invention, it being understood that they are in no way intended as limiting the scope thereof.

### Example 1: Preparation of 4'-benzyloxy-3'-benzylamino-3-hydroxyflavone

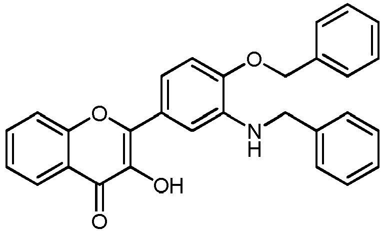

A suspension of 4-benzyloxy-3-benzylaminobenzaldehyde (57.2 mmol) and 2-hydroxyacetophenone (58.1 mmol) in ethanol (120 mL) and dioxane (75 mL) was cooled to 10°C, potassium hydroxide solution (40% w/v in H₂O, 45 mL) was added dropwise, then the mixture was stirred at room temperature for 6 d. The resulting red solution was diluted with dichloromethane (200 mL), washed with distilled water (4 × 100 mL) then the organic layer was dried (MgSO₄) and concentrated to give a brown residue. This residue was redissolved in dioxane (200 mL) and ethanol (450 mL) and treated with 2 M NaOH (140 mL) then the solution cooled to 0°C where H₂O₂ (30% w/v, 40 mL) was added dropwise. The reaction mixture was stirred at 0°C for 2 h then at room temperature for a further 17 h. The resulting yellow suspension was acidified with 2 M HCl (40 mL), filtered and the solid washed with ethanol. The crude solid was recrystallised from hot EtOAc to give the flavonol as a pale yellow fluffy solid. The filtrates from the crude and recrystallized materials were combined, concentrated under reduced pressure and a second crop of the flavonol extracted with EtOAc (120 mL). The organic extract was dried (MgSO₄), filtered and the filtrate concentrated and crystallized from EtOAc/petroleum spirits to give a second crop of the pure 3'-benzyloxy-4'-benzylamino-3-hydroxyflavone (61% yield).

### Example 2: Preparation of 3,4'-dihydroxy-3'-aminoflavone (90) - Method A

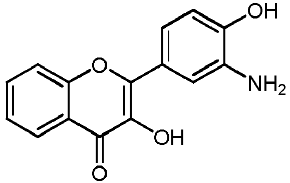

A mixture of 3'-benzyloxy-4'-benzylamino-3-hydroxyflavone (3.16 mmol) and Pd(OH)₂ (107 mg) in 9:1 THF:EtOH containing 0.05% acetic acid (50.0 mL) was treated with H₂ under high pressure (40 psi) for 5 h. The reaction mixture was filtered (Celite) and concentrated to give a dark green solid. The green residue was purified by flash chromatography (30 - 90% THF/toluene +1% acetic acid) followed by crystallization from THF/petroleum spirits to yield the pure 3,3'-dihydroxy-3'-aminoflavone as a pale brown solid (56%).

### Example 3: Preparation of 3,4'-dihydroxy-3'-aminoflavone (90) - Method B

An alternative approach to the synthesis of 3,4'-dihydroxy-3'-aminoflavone was also developed as shown in Scheme 1.

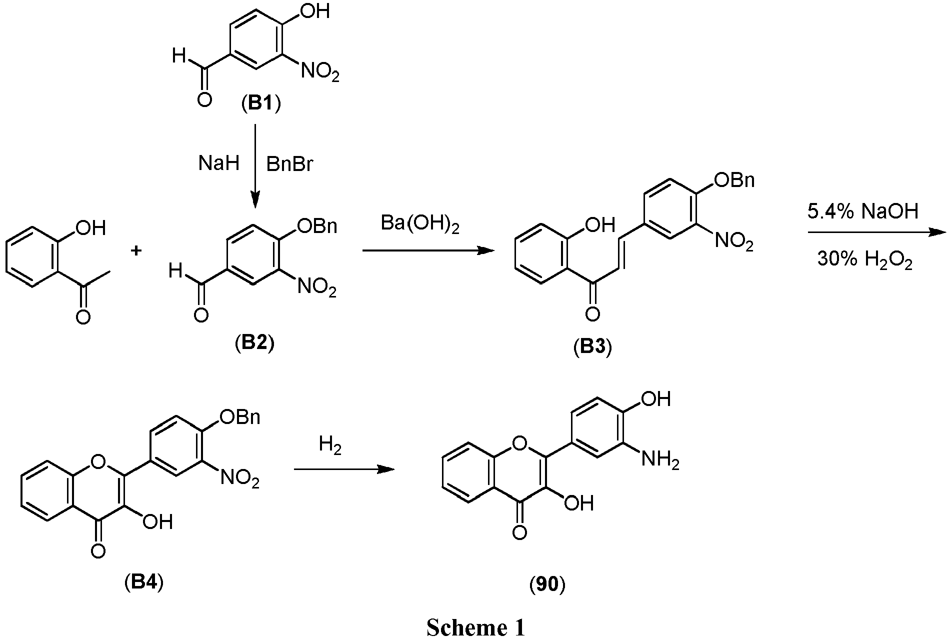

To a solution of (B-1) (5 g, 30 mmol) in dry dimethylacetamide (130 mL) at 0°C was added sodium hydride (60% dispersion in oil, 1.4 g, 36 mmol) in portions and the mixture was stirred at 0°C for 30 minutes. Benzyl bromide (5.34 g, 31.5 mmol) was added dropwise and the mixture was stirred at 0°C for 30 minutes, then at room temperature for 30 min and then heated at 70°C for 2 hours. TLC analysis showed the starting materials were consumed. The mixture was poured into ice/water (1 L) and stirred for 30 minutes. The precipitate was collected by filtration, washed with water and dried to give the product as a yellow solid (6.6 g, 86%). ¹H-NMR (400 MHz, d6-DMSO) δ (ppm): 9.91 (s, 1H), 8.41 (s, 1H), 8.15 (d, J=9.2 Hz, 1H), 7.63 (d, J=8.8 Hz, 1H), 7.48-7.36 (m, 5H), 5.41 (s, 2H).

A mixture of 2-hydroxyacetophenone (4.28 g, 31.5 mmol), B-2 (8.1 g, 31.5 mmol) and Ba(OH)₂ (10.8 g, 63 mmol) in methanol (400 mL) was stirred at 40°C overnight. TLC analysis showed that the starting materials were mostly consumed. The solvent was removed and the residue was diluted with water and neutralized with 1 M HCl to produce a solid. The solid was collected by filtration and washed with ethanol (300 mL) to give the product, B-3, as a yellow solid (6.2 g, 53%). ¹H-NMR (400 MHz, d₆-DMSO) δ (ppm): 12.6 (br s, 1H), 8.52 (s, 1H), 8.25 (d, J=7.2 Hz, 1H), 8.15 (d, J=7.2 Hz, 1H), 8.04 (d, J=15.6 Hz, 1H), 7.83 (d, J=15.6 Hz, 1H), 7.60-7.36 (m, 7H), 7.01 (m, 2H), 5.38 (s, 2H).

To a stirred solution of B-3 (6.2 g, 16.5 mmol) in methanol (220 mL) at 0°C was added 5.4% NaOH (52.8 mL) followed by 30% H₂O₂ (6.1 mL) dropwise and the mixture was stirred at 0 °C for 3 hours then allowed to warm to rt and stirred for a further 5 hours. The mixture was neutralized with 2 M HCl and the resulting precipitate was collected by filtration. The solid was washed with water, ethanol (200 mL) and ethanol:DCM (1:1, 40 mL) to give the product as a yellow solid (2.7 g, 42%). LC-MS : [M+H]⁺=390.1, [M+Na]⁺=412.0.

Compound B-4 (2.7 g, 6.9 mmol) was dissolved in methanol (180 mL), 10% Pd/C (200 mg) was added and the mixture was stirred under a hydrogen atmosphere at room temperature overnight. The mixture was filtered and the filtrate was concentrated under reduced pressure. The residue was washed with DCM:methanol (30:1, 100 mL x 2) to give the product, 3,4'-dihydroxy-3'-aminoflavone, (**90**) as a yellow solid (600 mg, 32%). LC-MS :[M+H]⁺=270.1, [M+Na]⁺=292.0; ¹H-NMR (400 MHz, d₆-DMSO) δ (ppm): 9.92 (br s, 1H), 9.16 (br s, 1H), 8.09 (dd, J=8.0, 1.2 Hz, 1H), 7.78 (m, 1H), 7.68 (d, J=8.4 Hz 1H), 7.53 (d, J=2.0 Hz, 1H), 7.47-7.41 (m, 2H), 6.81 (d, J=8.4 Hz, 1H), 4.76 (br s, 2H).

### Example 4: Preparation of 3,3'-dihydroxy-4'-aminoflavone (91)

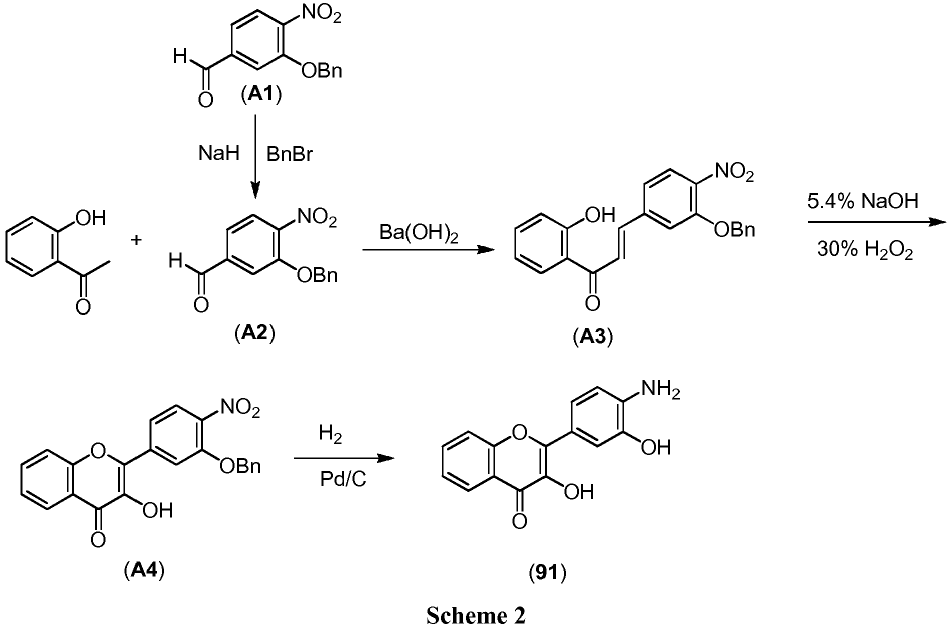

To a solution of A-1 (5 g, 30 mmol) in dry dimethylacetamide (130 mL) was added sodium hydride (60% dispersion in oil, 1.4 g, 36 mmol) in portions at 0°C. The mixture was stirred for 30 minutes at 0°C and then benzyl bromide (5.34 g, 31.5 mmol) was added dropwise. Stirring was continued at 0°C for 30 min then at room temperature for 30 min followed by heating at 70°C for 2 h. TLC analysis showed the starting materials were consumed. The mixture was poured into ice/water (1 L), stirred for 30 minutes and extracted with EtOAc (300ml x 3). The combined organic layers were washed with brine and dried over Na₂SO₄. The solvent was removed to give the product, A-2, as a brown solid (6.2 g, 81 %). ¹H-NMR (400 MHz, d₆-DMSO) δ (ppm): 10.0 (s, 1H), 8.05 (d, J=8.0Hz, 1H), 7.92 (s, 1H), 7.67 (d, J=8.0Hz, 1H), 7.47-7.35 (m, 5H), 5.40 (s, 2H).

A mixture of 2-hydroxyacetophenone (258 mg, 1.9 mmol), A-2 (500mg, 1.9 mmol) and Ba(OH)₂ (651 mg, 3.8 mmol) in methanol (80 mL) was heated at 40°C for 6 hours. TLC analysis showed the starting materials were mostly consumed. The solvent was removed and the residue was diluted with water and neutralized with 1 M HCl to give a solid. The solid was collected by filtration and washed with ethanol (30 mL) to give the product, A-3, as a yellow solid (500 mg, 70%). ¹H-NMR (400 MHz, d₆-DMSO) δ (ppm): 12.3 (s, 1H), 8.25 (d, J=7.6 Hz, 1H), 8.13 (d, J=15.6 Hz, 1H), 7.97-7.95 (m,2H), 7.81 (d, J=15.6 Hz, 1H), 7.65-7.58 (m, 2H), 7.50-7.34 (m, 5H), 7.06-7.34 (m, 2H), 5.39 (s, 2H).

To a stirred solution of A-3 (100 mg, 0.267 mmol) in methanol (10 mL) at 0 °C was added 5.4% NaOH (1 mL) followed by 30% H2O2 (0.2 mL) dropwise. The resulting mixture was stirred at 0 °C for 3 hours and then allowed to warm to rt and stirred for a further 5 hours. The reaction mixture was neutralized with 2 M HCl and the resulting precipitate was collected by filtration. The solid was washed with DCM to give A-4 (32 mg, 31%) as a yellow solid. LC-MS :[M+H]⁺=390.1, [M+Na]⁺=412.0; 1H-NMR (400 MHz, d₆-DMSO) δ (ppm): 8.20 (s, 1H), 8.13 (d, J=8.0 Hz, 1H), 8.08 (d, J=8.4 Hz, 1H), 8.00 (d, J=8.0 Hz, 1H), 7.88-7.81 (m, 2H), 7.54-7.36 (m, 6H), 5.42 (s, 2H).

Compound A-4 (1.1 g, 2.8 mmol) was dissolved in methanol (100 mL), 10% Pd/C (100 mg) was added and the mixture was stirred under a hydrogen atmosphere at room temperature overnight. The mixture was filtered and the filtrate was concentrated under reduced pressure. The residue was purified by silica gel chromatography (DCM:Methanol=70:1) to give the product as a yellow solid, 3,3'-dihydroxy-4'-aminoflavone (**91**) (310 mg, 41%). LC-MS :[M+H]⁺=270.1, [M+Na]⁺=292.0; ¹H-NMR (400 MHz, d₆-DMSO) δ (ppm): 9.52 (s, 1H), 9.11 (s, 1H), 8.07 (d, J=7.6 Hz, 1H), 7.75 (m, 1H), 7.68-7.66 (m, 2H), 7.60 (d, J=8.4 Hz, 1H), 7.44 (m, 1H), 6.73 (d, J=8.4 Hz, 1H), 5.25 (s, 2H).

### Example 5: Preparation of 2-(3',4'-dibenzyloxyphenyl)- 3-hydroxy-4H-pyrano [2,3-b] pyridine-4-one

A suspension of 3,4-dibenzyloxy-benzaldehyde (57.2 mmol) and 2-hydroxy-3-pyridylcarboxaldehyde (58.1 mmol) in ethanol (120 mL) and dioxane (75 mL) was cooled to 10 °C, potassium hydroxide solution (40% w/v in H₂O, 45 mL) was added dropwise, then the mixture was stirred at room temperature for 6 d. The resulting red solution was diluted with dichloromethane (200 mL), washed with distilled water (4 × 100 mL) then the organic layer was dried (MgSO₄) and concentrated to give a brown residue. This residue was redissolved in dioxane (200 mL) and ethanol (450 mL) and treated with 2 M NaOH (140 mL) then the solution cooled to 0°C where H₂O₂ (30% w/v, 40 mL) was added dropwise. The reaction mixture was stirred at 0°C for 2 h then at room temperature for a further 17 h. The resulting yellow suspension was acidified with 2 M HCl (40 mL), filtered and the solid washed with ethanol. The crude solid was recrystallised from hot EtOAc to give the flavonol as a pale yellow fluffy solid. The filtrates from the crude and recrystallized materials were combined, concentrated under reduced pressure and a second crop of the flavonol extracted with EtOAc (120 mL). The organic extract was dried (MgSO₄), filtered and the filtrate concentrated and crystallized from EtOAc/petroleum spirits to give a second crop of the pure 3'-benzyloxy-4'-benzylamino-3-hydroxyflavone (61% yield).

### Example 6: Preparation of 2-(3',4'-dihydroxyphenyl)- 3-hydroxy-4H-pyrano[2,3-b]ridine-4-one

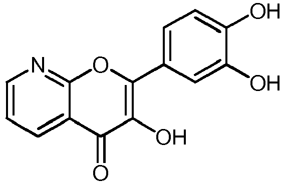

A mixture of 2-(3',4'-Dibenzyloxyphenyl)-3-hydroxy-4H-pyrano[2,3-b]pyridine-4-one (3.16 mmol) and Pd(OH)₂ (107 mg) in 9:1 THF:EtOH containing 0.05% acetic acid (50.0 mL) was treated with H₂ under high pressure (40 psi) for 5 h. The reaction mixture was filtered (Celite) and concentrated to give a dark green solid. The green residue was purified by flash chromatography (30 - 90% THF/toluene +1% acetic acid) followed by crystallization from THF/petroleum spirits to yield the pure 2-(3',4'-dihydroxyphenyl)-3-hydroxy-4H-pyrano[2,3-b]pyridine-4-one as a pale brown solid (56%).

### Example 7: Preparation of 2-(3',4'-dibenzyloxyphenyl)- 3-hydroxy-3H-pyrano [2,3-b] pyridine-4-one

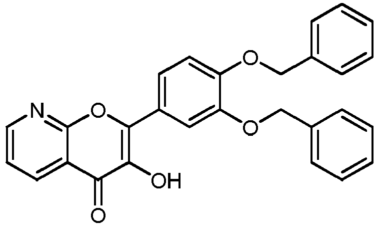

A suspension of 3,4-dibenzyloxy-benzaldehyde (57.2 mmol) and 3-hydroxy-4-pyridylcarboxaldehyde (58.1 mmol) in ethanol (120 mL) and dioxane (75 mL) was cooled to 10 °C, potassium hydroxide solution (40% w/v in H₂O, 45 mL) was added dropwise, then the mixture was stirred at room temperature for 6 d. The resulting red solution was diluted with dichloromethane (200 mL), washed with distilled water (4 × 100 mL) then the organic layer was dried (MgSO₄) and concentrated to give a brown residue. This residue was redissolved in dioxane (200 mL) and ethanol (450 mL) and treated with 2 M NaOH (140 mL) then the solution cooled to 0°C where H₂O₂ (30% w/v, 40 mL) was added dropwise. The reaction mixture was stirred at 0°C for 2 h then at room temperature for a further 17 h. The resulting yellow suspension was acidified with 2 M HCl (40 mL), filtered and the solid washed with ethanol. The crude solid was recrystallised from hot EtOAc to give the flavonol as a pale yellow fluffy solid. The filtrates from the crude and recrystallized materials were combined, concentrated under reduced pressure and a second crop of the flavonol extracted with EtOAc (120 mL). The organic extract was dried (MgSO₄), filtered and the filtrate concentrated and crystallized from EtOAc/petroleum spirits to give a second crop of the pure 3'-benzyloxy-4'-benzylamino-3-hydroxyflavone (61% yield).

### Example 8: Preparation of 2-(3',4'-dihydroxyphenyl)- 3-hydroxy-3H-pyrano[2,3-b]pyridine-4-one

A mixture of 2-(3',4'-dibenzyloxyphenyl)-3'-hydroxy-3H-pyrano[2,3-b]pyridine-4-one (3.16 mmol) and Pd(OH)₂ (107 mg) in 9:1 THF:EtOH containing 0.05% acetic acid (50.0 mL) was treated with H₂ under high pressure (40 psi) for 5 h. The reaction mixture was filtered (Celite) and concentrated to give a dark green solid. The green residue was purified by flash chromatography (30 - 90% THF/toluene +1% acetic acid) followed by crystallization from THF/petroleum spirits to yield the pure product as a pale brown solid (56%).

### Example 9: MTT Assay Protocols

MTT assays on the present compounds were performed according to the following steps:
i) Culturing cells in a 96-well plate under desired conditions;
ii) Prepare a 12mM MTT stock solution by dissolving 5mg MTT (Sigma M5655) in 1ml of sterile PBS. Mix by vortexing until dissolved;
iii) Prepare a 10ml MTT working solution by adding 1ml MTT stock solution to 9ml starvation medium. Keep covered;
iv) Remove the medium and replace it with 100µl of MTT working solution in each well (including a negative control with no cells present in the well);
v) Incubate the plate in dark at 37°C, 6 % CO₂ for 4hrs;
vi) Remove medium from wells and add 100µl isopropanol to each well (also to the negative control);
vii) Place the plate in the incubator for 20 min or until blue formazan crystals have been dissolved in isopropanol;
viii) Read absorbance at 570nm and 690nm

## Claims

1. A compound of the general formula (I): 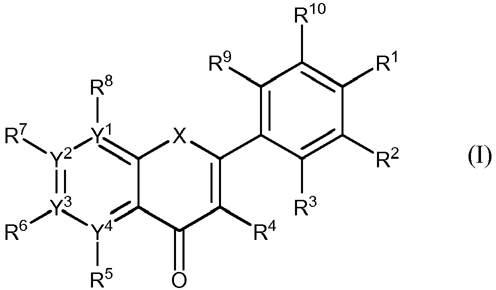 wherein
X represents an NH, S or O;
Y¹, Y², Y³, Y⁴ are independently selected from C or N;
R¹, R² and R⁴ are independently selected from the group comprising hydroxyl, haloalkyl, haloalkoxy, heterocyclic and substituted heterocyclic, -NH₂, -NH(C₁-C₆ alkyl) and - N(C₁-C₆ alkyl)₂;
R³, R⁹ and R¹⁰ are independently selected from the group comprising H, halogen and C₁-C₆ alkyl;
R⁵, R⁶, R⁷ and R⁸ where present are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and -N(C₁-C₆ alkyl)₂;
with the *proviso* that the compound is not selected from the group comprising:
2-(3,4-dihydroxyphenyl)-3,5,7-trihydroxy-4H-1-benzothiopyran-4-one;
2-(2,3-dihydroxyphenyl)-5,8-dihydroxy-4H-1-benzothiopyran-4-one;
2-(3,4-dihydroxyphenyl)-3-hydroxy-4(1H)-quino linone;
2-(3,4-dihydroxyphenyl)-3,7-dihydroxy-4(1H)-quinolinone, hydrobromide;
2-(3 ,4-dihydroxyphenyl)-3,7-dihydroxy-4(1H)-quinolinone;
2-(3,4-dihydroxyphenyl)-3,6-dihydroxy-4(1H)-quinolinone;
2-(3,4-dihydroxyphenyl)-3,5-dihydroxy-4(1H)-quinolinone, hydrobromide;
2-(3,4-dihydroxyphenyl)-3,5-dihydroxy-4(1H)-quinolinone;
2-(3,4-dihydroxyphenyl)-3,6,7-trihydroxy-4(1H)-quinolinone;
2-(3,4-dihydroxyphenyl)-3,5,7-trihydroxy-4(1H)-quinolinone;
2-(3,4-dihydroxyphenyl)-3-hydroxy-5,7-dimethyl-4(1H)-quinolinone, hydrobromide;
2-(3,4-dihydroxyphenyl)-3-hydroxy-5,7-dimethyl-4(1H)-quinolinone;
2-(3,4-dihydroxyphenyl)-3-hydroxy-7-methoxy-4(1H)-quinolinone;
2-(3,4-dihydroxyphenyl)-3-hydroxy-6-methoxy-4(1H)-quino linone;
2-(3,4-dihydroxyphenyl)-3,6,7,8-tetrahydroxy-4(1H)-quinolinone;
2-(3,4-dihydroxyphenyl)-3-hydroxy-5-methoxy-4(1H)-quinolinone;
2-(3,4-dihydroxyphenyl)-3-hydroxy-6,7-dimethoxy-4(1H)-quinolinone;
2-(3-amino-4-hydroxyphenyl)-3,5,7-trihydroxy-4H-1-benzopyran-4-one;
N-[2-(3-amino-4-hydroxyphenyl)-3-hydroxy-6-methyl-4-oxo-4H-chromen-8-yl]-acetamide;
N-[3-hydroxy-2-[4-hydroxy-3-[91-methylethyl)amino]phenyl]6-methyl-4-oxo-4H-1-benzopyran-8-yl]-acetamide;
N-[2-hydroxy-5-(3-hydroxy-6-methyl-4-oxo-4H-1-benzopyran-2-yl)phenyl]-acetamide;
2-(3-amino-4-hydroxyphenyl)-3-hydroxy-6-methyl-4H-1-benzopyran-4-one;
8-amino-2-(3-amino-4-hydroxyphenyl)-3-hydroxy-6-methyl-4H-1-benzopyran-4-one;
N-[2-Hydroxy-5-(3-hydroxy-6-methyl-4-oxo-4H-1-benzopyran-2-yl)phenyl]-acetamide;
N-[2-(3,4-dihydroxyphenyl)-3-hydroxy-4-oxo-4H-1-benzopyran-6-yl]-acetamide;
N-[2-(3-amino-4-hydroxyphenyl)-3-hydroxy-6-methyl-4-oxo-4H-1-benzopyran-8-yl]-acetamide;
6-acetamido-3-hydroxy-4'-methoxy-flavone;
3,3'-dihydroxy,6,7-dimethyl,4'-(pyrrolidine-1-yl)-flavone; and
2-(3-amino-4-hydrophenyl)-3,5,7-trihydroxy-4H-1-benzopyran-4-one.

2. The compound of claim 1, wherein the haloalkyl is selected from the group comprising -CF₂H, -CH₂F, -CH₂CF₂H, and -CF₂CF₂H.

3. The compound of claim 1 or claim 2, where in the haloalkoxy is selected from the group comprising -OCF₂H, -OCH₂F, -OCH₂CF₂H, -OCF₂CF₂H.

4. The compound of any preceding claim, wherein the heterocyclic is selected from the group comprising

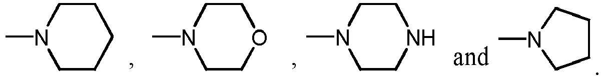

5. The compound of any preceding claim, wherein the -NH(C₁-C₆alkyl) group is selected from the group comprising -NHCH₃, -NHCH₂CH₃ and -NHCH₂CH₂CH₃.

6. The compound of any preceding claim, wherein the N(C₁-C₆alkyl)₂ is selected from-N(CH₃)₂, -N(CH₂CH₃)₂, and -N(CH₂CH₂CH₃)₂.

7. The compound of any preceding claim, wherein:
X is O;
Y¹, Y², Y³, Y⁴ are C;
R⁵, R⁶, R⁷ and R⁸ are H; or
X is O;
Y¹, Y³, Y⁴ are C;
Y² is N;
R⁵, R⁶ and R⁸ are H;
R⁷ is absent; or
X is O;
Y¹, Y², Y⁴ are C;
Y³ is N;
R⁵, R⁷ and R⁸ are H;
R⁶ is absent; or
X is NH;
Y¹, Y², Y³ and Y⁴ are C;
R⁵, R⁶, R⁷ and R⁸ are H; or
X is S;
Y¹, Y², Y³ and Y⁴ are C;
R⁵, R⁶, R⁷ and R⁸ are H; and
R¹ is OH, R² is NH₂ and R⁴ is OH; or
R¹ is NH₂, R² is OH and R⁴ is OH; or
R¹ is OH, R² is NHCH₃ and R⁴ is OH; or
R¹ is NHCH₃, R² is OH and R⁴ is OH; or
R¹ is CF₂H, R² is OH and R⁴ is OH; or
R¹ is OH, R² is CF₂H and R⁴ is OH; or
R¹ is CF₂H, R² is CF₂H and R⁴ is OH; or
R¹ is OH, R² is OCF₂H and R⁴ is OH; or
R¹ is OCF₂H, R² is OH and R⁴ is OH; or
R¹ is OCF₂H, R² is OCF₂H and R⁴ is OH; or
R¹ is OCF₂H, R² is CF₂H and R⁴ is OH; or
R¹ is CF₂H, R² is OCF₂H and R⁴ is OH; or
R¹ is OH, R² is NH₂ and R⁴ is OH; or
R¹ is OH, R² is OH and R⁴ is NH₂; or
R¹ is NH₂, R² is OH and R⁴ is NH₂; or
R¹ is OH, R² is NHCH₃ and R⁴ is NH₂; or
R¹ is NHCH₃, R² is OH and R⁴ is NH₂; or
R¹ is CF₂H, R² is OH and R⁴ is NH₂; or
R¹ is OH, R² is CF₂H and R⁴ is NH₂; or
R¹ is CF₂H, R² is CF₂H and R⁴ is NH₂; or
R¹ is OH, R² is OCF₂H and R⁴ is NH₂; or
R¹ is OCF₂H, R² is OH and R⁴ is NH₂; or
R¹ is OCF₂H, R² is OCF₂H and R⁴ is NH₂; or
R¹ is OCF₂H, R² is CF₂H and R⁴ is NH₂; or
R¹ is CF₂H, R² is OCF₂H and R⁴ is NH₂; or
R¹ is OH, R² is OH and R⁴ is NHCH₃; or
R¹ is OH, R² is NH₂ and R⁴ is NHCH₃; or
R¹ is NH₂, R² is OH and R⁴ is NHCH₃; or
R¹ is CF₂H, R² is OH and R⁴ is NHCH₃; or
R¹ is OH, R² is CF₂H and R⁴ is NHCH₃; or
R¹ is OCF₂H, R² is OH and R⁴ is NHCH₃; or
R¹ is OH, R² is OCF₂H and R⁴ is NHCH₃; or
R¹ is OH, R² is OH and R⁴ is NHCH₃; or
R¹ is NH₂, R² is OH and R⁴ is NHCH₃; or
R¹ is OH, R² is NHCH₃ and R⁴ is NHCH₃; or
R¹ is NHCH₃, R² is OH and R⁴ is NHCH₃; or
R¹ is CF₂H, R² is OH and R⁴ is NHCH₃; or
R¹ is OH, R² is CF₂H and R⁴ is NHCH₃; or
R¹ is CF₂H, R² is CF₂H and R⁴ is NHCH₃; or
R¹ is OH, R² is OCF₂H and R⁴ is NHCH₃; or
R¹ is OCF₂H, R² is OH and R⁴ is NHCH₃; or
R¹ is OCF₂H, R² is OCF₂H and R⁴ is NHCH₃; or
R¹ is OCF₂H, R² is CF₂H and R⁴ is NHCH₃; or
R¹ is CF₂H, R² is OCF₂H and R⁴ is NHCH₃; or
(R₁ is OH, R₂ is 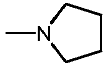 and R₄ is OH; or
R₁ is 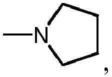 R₂ is OH and R₄ is OH.

8. A pharmaceutical and/or veterinary composition comprising a pharmaceutically and/or veterinarily acceptable carrier or diluent together with a compound according to any one of claims 1 to 7.

9. A compound of formula (I) for use in the prevention and /or mitigation of a disease caused by cell apoptosis and / or cell necrosis in a subject; 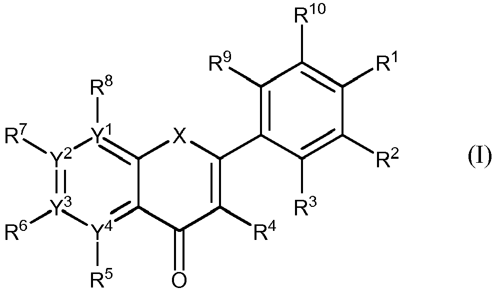 wherein
X represents an NH, S or O;
Y¹, Y², Y³, Y⁴ are independently selected from C or N;
R¹, R² and R⁴ are independently selected from the group comprising hydroxyl, haloalkyl, haloalkoxy, heterocyclic, substituted heterocyclic, -NH₂, -NH(C₁-C₆ alkyl) and - N(C₁-C₆ alkyl)₂;
R³, R⁹ and R¹⁰ are independently selected from the group comprising H, halogen and C₁-C₆ alkyl;
R⁵, R⁶, R⁷ and R⁸ where present are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and -N(C₁-C₆ alkyl)₂.

10. A compound of formula (I) for use in the prevention and/or treatment of a disease(s) in a subject associated with the presence of reactive oxidative species (ROS): 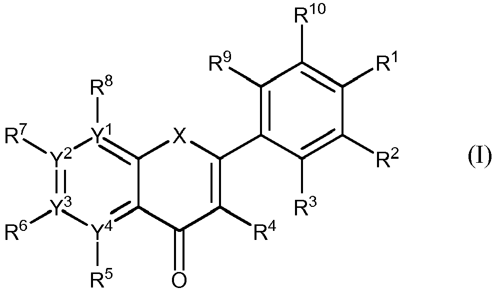 wherein
X represents an NH, S or O;
Y¹, Y², Y³, Y⁴ are independently selected from C or N;
R¹, R² and R⁴ are independently selected from the group comprising hydroxyl, haloalkyl, haloalkoxy, heterocyclic, substituted heterocyclic, -NH₂, -NH(C₁-C₆ alkyl) and - N(C₁-C₆ alkyl)₂;
R³, R⁹ and R¹⁰ are independently selected from the group comprising H, halogen and C₁-C₆ alkyl;
R⁵, R⁶, R⁷ and R⁸ where present are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and -N(C₁-C₆ alkyl)₂.

11. A compound of formula (I) for use in the prevention and /or mitigation of ischaemia, reperfusion injury, cerebrovascular disease, pulmonary vascular disease, atherosclerosis, stroke, myocardial infarction, artery disease, congestive heart disease, coronary disease, peripheral vascular disease, diabetes, hypertension, migraine, chronic obstructive pulmonary disease or retinal vascular disease: 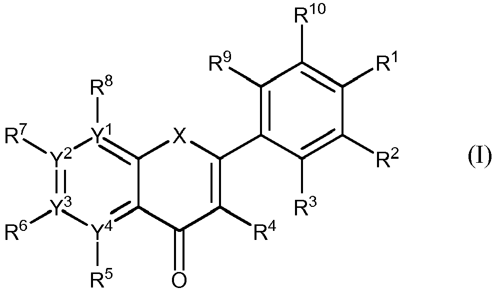 wherein
X represents an NH, S or O;
Y¹, Y², Y³, Y⁴ are independently selected from C or N;
R¹, R² and R⁴ are independently selected from the group comprising hydroxyl, haloalkyl, haloalkoxy, heterocyclic, substituted heterocyclic, -NH₂, -NH(C₁-C₆ alkyl) and - N(C₁-C₆ alkyl)₂;
R³, R⁹ and R¹⁰ are independently selected from the group comprising H, halogen and C₁-C₆ alkyl;
R⁵, R⁶, R⁷ and R⁸ where present are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and -N(C₁-C₆ alkyl)₂.

12. A compound according to claim 9 or claim 10 for use in the prevention and /or mitigation of a disease caused by cell apoptosis and / or cell necrosis in a subject, and/or for use in the prevention and/or treatment of a disease(s) in a subject associated with the presence of reactive oxidative species (ROS) for use in the treatment of reperfusion injury as a result of an acute or chronic condition, the subject suffering from mechanical trauma resulting from crush injury or surgery; ischaemia and/or reperfusion injury as a result of an acute or chronic condition; disease which is the result of vascular surgery or disease is the result of heart bypass and/or transplant surgery.

13. A compound of formula (I) for use in the prevention and /or mitigation of ischaemia or reperfusion injury: 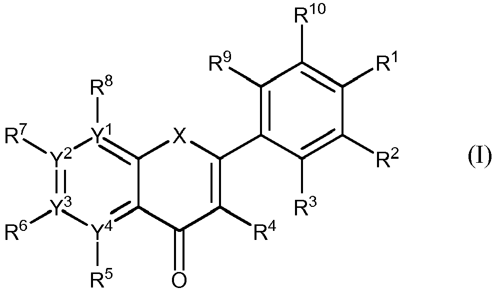 wherein
X represents an NH, S or O;
Y¹, Y², Y³, Y⁴ are independently selected from C or N;
R¹, R² and R⁴ are independently selected from the group comprising hydroxyl, haloalkyl, haloalkoxy, heterocyclic, substituted heterocyclic, -NH₂, -NH(C₁-C₆ alkyl) and - N(C₁-C₆ alkyl)₂;
R³, R⁹ and R¹⁰ are independently selected from the group comprising H, halogen and C₁-C₆alkyl;
R⁵, R⁶, R⁷ and R⁸ where present are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and -N(C₁-C₆ alkyl)₂.

14. A compound of formula (I) for use in ameliorating damage to a subject caused by the administration of a therapeutic agent: 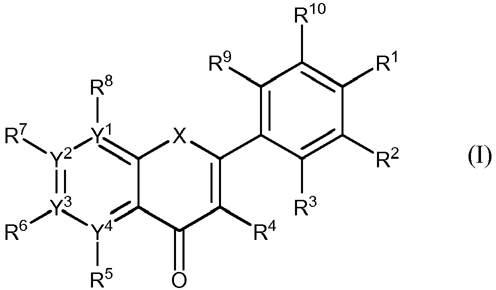 wherein
X represents an NH, S or O;
Y¹, Y², Y³, Y⁴ are independently selected from C or N;
R¹, R² and R⁴ are independently selected from the group comprising hydroxyl, haloalkyl, haloalkoxy, heterocyclic, substituted heterocyclic, -NH₂, -NH(C₁-C₆ alkyl) and - N(C₁-C₆ alkyl)₂;
R³, R⁹ and R¹⁰ are independently selected from the group comprising H, halogen and C₁-C₆ alkyl;
R⁵, R⁶, R⁷ and R⁸ where present are H; or
a pharmaceutically acceptable salt, hydrate and/or solvate thereof;
with the *proviso* that at least one of R¹, R² and R⁴ is selected from the group comprising haloalkyl, haloalkoxy, heterocyclic, substituted heterocyclic, -NH₂, -NH(C₁-C₆ alkyl) and -N(C₁-C₆ alkyl)₂.

## Patentansprüche

1. Eine Verbindung der allgemeinen Formel (I): 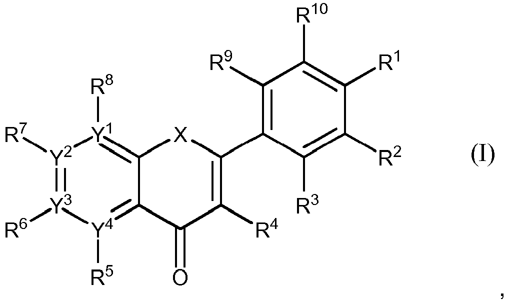 wobei
X NH, S oder O bedeutet,
Y¹, Y², Y³, Y⁴ unabhängig ausgewählt sind aus C oder N,
R¹, R² und R⁴ unabhängig ausgewählt sind aus der Gruppe, die Hydroxyl, Halogenalkyl, Halogenalkoxy, Heterocyclus und substituierten Heterocyclus, -NH₂, -NH(C₁-C₆-Alkyl) und -N(C₁-C₆-Alkyl)₂ umfasst,
R³, R⁹ und R¹⁰ unabhängig ausgewählt sind aus der Gruppe, die H, Halogen und C₁-C₆-Alkyl umfasst,
R⁵, R⁶, R⁷ und R⁸, wo vorhanden, H sind, oder
ein pharmazeutisch annehmbares Salz, Hydrat und/oder Solvat davon,
mit der Maßgabe, dass wenigstens eines von R¹, R² und R⁴ ausgewählt ist aus der Gruppe, die Halogenalkyl, Halogenalkoxy, Heterocyclus, substituierten Heterocyclus, -NH₂, -NH(C₁-C₆-Alkyl) und -N(C₁-C₆-Alkyl)₂ umfasst,
mit der Maßgabe, dass die Verbindung nicht ausgewählt ist aus der Gruppe, die umfasst:
2-(3,4-Dihydroxyphenyl)-3,5,7-trihydroxy-4H-1-benzothiopyran-4-on,
2-(2,3-Dihydroxyphenyl)-5,8-dihydroxy-4H-1-benzothiopyran-4-on,
2-(3,4-Dihydroxyphenyl)-3-hydroxy-4(1H)-chinolinon,
2-(3,4-Dihydroxyphenyl)-3,7-dihydroxy-4(1H)-chinolinon-Hydrobromid,
2-(3,4-Dihydroxyphenyl)-3,7-dihydroxy-4(1H)-chinolinon,
2-(3,4-Dihydroxyphenyl)-3,6-dihydroxy-4(1H)-chinolinon,
2-(3,4-Dihydroxyphenyl)-3,5-dihydroxy-4(1H)-chinolinon-Hydrobromid,
2-(3,4-Dihydroxyphenyl)-3,5-dihydroxy-4(1H)-chinolinon,
2-(3,4-Dihydroxyphenyl)-3,6,7-trihydroxy-4(1H)-chinolinon,
2-(3,4-Dihydroxyphenyl)-3,5,7-trihydroxy-4(1H)-chinolinon,
2-(3,4-Dihydroxyphenyl)-3-hydroxy-5,7-dimethyl-4(1H)-chinolinon-Hydrobromid,
2-(3,4-Dihydroxyphenyl)-3-hydroxy-5,7-dimethyl-4(1H)-chinolinon,
2-(3,4-Dihydroxyphenyl)-3-hydroxy-7-methoxy-4(1H)-chinolinon,
2-(3,4-Dihydroxyphenyl)-3-hydroxy-6-methoxy-4(1H)-chinolinon,
2-(3,4-Dihydroxyphenyl)-3,6,7,8-tetrahydroxy-4(1H)-chinolinon,
2-(3,4-Dihydroxyphenyl)-3-hydroxy-5-methoxy-4(1H)-chinolinon,
2-(3,4-Dihydroxyphenyl)-3-hydroxy-6,7-dimethoxy-4(1H)-chinolinon,
2-(3-Amino-4-hydroxyphenyl)-3,5,7-trihydroxy-4H-1-benzopyran-4-on,
N-[2-(3-Amino-4-hydroxyphenyl)-3-hydroxy-6-methyl-4-oxo-4H-chromen-8-yl]acetamid,
N-[3-Hydroxy-2-[4-hydroxy-3-[(1-methylethyl)amino]phenyl]-6-methyl-4-oxo-4H-1-benzopyran-8-yl]acetamid,
N-[2-Hydroxy-5-(3-hydroxy-6-methyl-4-oxo-4H-1-benzopyran-2-yl)-phenyl]acetamid,
2-(3-Amino-4-hydroxyphenyl)-3-hydroxy-6-methyl-4H-1-benzopyran-4-on,
8-Amino-2-(3-amino-4-hydroxyphenyl)-3-hydroxy-6-methyl-4H-1-benzopyran-4-on,
N-[2-Hydroxy-5-(3-hydroxy-6-methyl-4-oxo-4H-1-benzopyran-2-yl)phenyl]-acetamid,
N-[2-(3,4-Dihydroxyphenyl)-3-hydroxy-4-oxo-4H-1-benzopyran-6-yl]-acetamid,
N-[2-(3-Amino-4-hydroxyphenyl)-3-hydroxy-6-methyl-4-oxo-4H-1-benzopyran-8-yl]acetamid,
6-Acetamido-3-hydroxy-4'-methoxyflavon,
3,3'-Dihydroxy-6,7-dimethyl-4'-(pyrrolidin-1-yl)flavon und
2-(3-Amino-4-hydrophenyl)-3,5,7-trihydroxy-4H-1-benzopyran-4-on.

2. Die Verbindung nach Anspruch 1, wobei das Halogenalkyl ausgewählt ist aus der Gruppe, die -CF₂H, -CH₂F, -CH₂CF₂H und -CF₂CF₂H umfasst.

3. Die Verbindung nach Anspruch 1 oder Anspruch 2, wobei das Halogenalkoxy ausgewählt ist aus der Gruppe, die -OCF₂H, -OCH₂F, -OCH₂CF₂H, -OCF₂CF₂H umfasst.

4. Die Verbindung nach einem vorhergehenden Anspruch, wobei der Heterocyclus ausgewählt ist aus der Gruppe, die 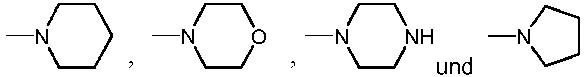 umfasst.

5. Die Verbindung nach einem vorhergehenden Anspruch, wobei die -NH(C₁-C₆-Alkyl)-Gruppe ausgewählt ist aus der Gruppe, die -NHCH₃, -NHCH₂CH₃ und -NHCH₂CH₂CH₃ umfasst.

6. Die Verbindung nach einem vorhergehenden Anspruch, wobei das N(C₁-C₆-Alkyl)₂ ausgewählt ist aus -N(CH₃)₂, -N(CH₂CH₃)₂ und -N(CH₂CH₂CH₃)₂.

7. Die Verbindung nach einem vorhergehenden Anspruch, wobei:
X O ist,
Y¹, Y², Y³, Y⁴ C sind,
R⁵, R⁶, R⁷ und R⁸ H sind, oder
X O ist,
Y¹, Y³, Y⁴ C sind,
Y² N ist,
R⁵, R⁶ und R⁸ H sind,
R⁷ fehlt, oder
X O ist,
Y¹, Y², Y⁴ C sind,
Y³ N ist,
R⁵, R⁷ und R⁸ H sind,
R⁶ fehlt, oder
X NH ist,
Y¹, Y², Y³ und Y⁴ C sind,
R⁵, R⁶, R⁷ und R⁸ H sind, oder
X S ist,
Y¹, Y², Y³ und Y⁴ C sind,
R⁵, R⁶, R⁷ und R⁸ H sind, und
R¹ OH ist, R² NH₂ ist und R⁴ OH ist, oder
R¹ NH₂ ist, R² OH ist und R⁴ OH ist, oder
R¹ OH ist, R² NHCH₃ ist und R⁴ OH ist, oder
R¹ NHCH₃ ist, R² OH ist und R⁴ OH ist, oder
R¹ CF₂H ist, R² OH ist und R⁴ OH ist, oder
R¹ OH ist, R² CF₂H ist und R⁴ OH ist, oder
R¹ CF₂H ist, R² CF₂H ist und R⁴ OH ist, oder
R¹ OH ist, R² OCF₂H ist und R⁴ OH ist, oder
R¹ OCF₂H ist, R² OH ist und R⁴ OH ist, oder
R¹ OCF₂H ist, R² OCF₂H ist und R⁴ OH ist, oder
R¹ OCF₂H ist, R² CF₂H ist und R⁴ OH ist, oder
R¹ CF₂H ist, R² OCF₂H ist und R⁴ OH ist, oder
R¹ OH ist, R² NH₂ ist und R⁴ OH ist, oder
R¹ OH ist, R² OH ist und R⁴ NH₂ ist, oder
R¹ NH₂ ist, R² OH ist und R⁴ NH₂ ist, oder
R¹ OH ist, R² NHCH₃ ist und R⁴ NH₂ ist, oder
R¹ NHCH₃ ist, R² OH ist und R⁴ NH₂ ist, oder
R¹ CF₂H ist, R² OH ist und R⁴ NH₂ ist, oder
R¹ OH ist, R² CF₂H ist und R⁴ NH₂ ist, oder
R¹ CF₂H ist, R² CF₂H ist und R⁴ NH₂ ist, oder
R¹ OH ist, R² OCF₂H ist und R⁴ NH₂ ist, oder
R¹ OCF₂H ist, R² OH ist und R⁴ NH₂ ist, oder
R¹ OCF₂H ist, R² OCF₂H ist und R⁴ NH₂ ist, oder
R¹ OCF₂H ist, R² CF₂H ist und R⁴ NH₂ ist, oder
R¹ CF₂H ist, R² OCF₂H ist und R⁴ NH₂ ist, oder
R¹ OH ist, R² OH ist und R⁴ NHCH₃ ist, oder
R¹ OH ist, R² NH₂ ist und R⁴ NHCH₃ ist, oder
R¹ NH₂ ist, R² OH ist und R⁴ NHCH₃ ist, oder
R¹ CF₂H ist, R² OH ist und R⁴ NHCH₃ ist, oder
R¹ OH ist, R² CF₂H ist und R⁴ NHCH₃ ist, oder
R¹ OCF₂H ist, R² OH ist und R⁴ NHCH₃ ist, oder
R¹ OH ist, R² OCF₂H ist und R⁴ NHCH₃ ist, oder
R¹ OH ist, R² OH ist und R⁴ NHCH₃ ist, oder
R¹ NH₂ ist, R² OH ist und R⁴ NHCH₃ ist, oder
R¹ OH ist, R² NHCH₃ ist und R⁴ NHCH₃ ist, oder
R¹ NHCH₃ ist, R² OH ist und R⁴ NHCH₃ ist, oder
R¹ CF₂H ist, R² OH ist und R⁴ NHCH₃ ist, oder
R¹ OH ist, R² CF₂H ist und R⁴ NHCH₃ ist, oder
R¹ CF₂H ist, R² CF₂H ist und R⁴ NHCH₃ ist, oder
R¹ OH ist, R² OCF₂H ist und R⁴ NHCH₃ ist, oder
R¹ OCF₂H ist, R² OH ist und R⁴ NHCH₃ ist, oder
R¹ OCF₂H ist, R² OCF₂H ist und R⁴ NHCH₃ ist, oder
R¹ OCF₂H ist, R² CF₂H ist und R⁴ NHCH₃ ist, oder
R¹ CF₂H ist, R² OCF₂H ist und R⁴ NHCH₃ ist, oder
(R₁ OH ist, R₂ 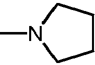 ist und R₄ OH ist, oder
R₁ 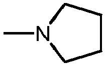 ist, R₂ OH ist und R₄ OH ist.

8. Eine pharmazeutische und/oder veterinärmedizinische Zusammensetzung, die einen pharmazeutisch und/oder veterinärmedizinisch annehmbaren Träger oder ein pharmazeutisch und/oder veterinärmedizinisch annehmbares Verdünnungsmittel zusammen mit einer Verbindung gemäß einem der Ansprüche 1 bis 7 umfasst.

9. Eine Verbindung der Formel (I) zur Verwendung bei der Prävention und/oder Linderung einer Erkrankung, die durch Zellapoptose und/oder Zellnekrose hervorgerufen wird, bei einem Subjekt, 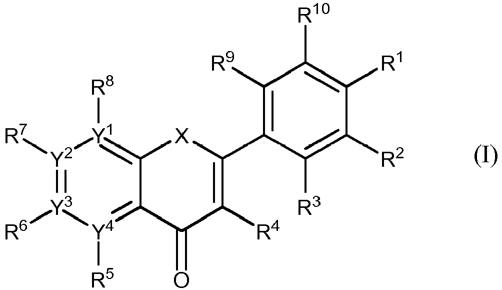 wobei
X NH, S oder O bedeutet,
Y¹, Y², Y³, Y⁴ unabhängig ausgewählt sind aus C oder N,
R¹, R² und R⁴ unabhängig ausgewählt sind aus der Gruppe, die Hydroxyl, Halogenalkyl, Halogenalkoxy, Heterocyclus, substituierten Heterocyclus, -NH₂, -NH(C₁-C₆-Alkyl) und -N(C₁-C₆-Alkyl)₂ umfasst,
R³, R⁹ und R¹⁰ unabhängig ausgewählt sind aus der Gruppe, die H, Halogen und C₁-C₆-Alkyl umfasst,
R⁵, R⁶, R⁷ und R⁸, wo vorhanden, H sind, oder
ein pharmazeutisch annehmbares Salz, Hydrat und/oder Solvat davon,
mit der Maßgabe, dass wenigstens eines von R¹, R² und R⁴ ausgewählt ist aus der Gruppe, die Halogenalkyl, Halogenalkoxy, Heterocyclus, substituierten Heterocyclus, -NH₂, -NH(C₁-C₆-Alkyl) und -N(C₁-C₆-Alkyl)₂ umfasst.

10. Eine Verbindung der Formel (I) zur Verwendung bei der Prävention und/oder Behandlung einer oder mehrerer Erkrankungen, die mit dem Vorhandensein reaktiver oxidativer Spezies (ROS) assoziiert ist/sind, bei einem Subjekt, 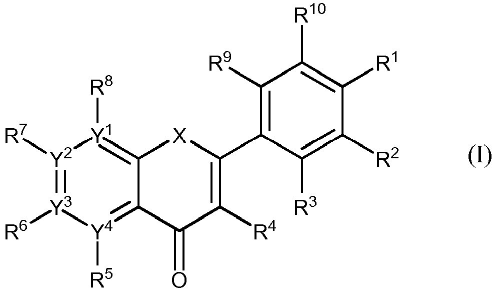 wobei
X NH, S oder O bedeutet,
Y¹, Y², Y³, Y⁴ unabhängig ausgewählt sind aus C oder N,
R¹, R² und R⁴ unabhängig ausgewählt sind aus der Gruppe, die Hydroxyl, Halogenalkyl, Halogenalkoxy, Heterocyclus, substituierten Heterocyclus, -NH₂, -NH(C₁-C₆-Alkyl) und -N(C₁-C₆-Alkyl)₂ umfasst,
R³, R⁹ und R¹⁰ unabhängig ausgewählt sind aus der Gruppe, die H, Halogen und C₁-C₆-Alkyl umfasst,
R⁵, R⁶, R⁷ und R⁸, wo vorhanden, H sind, oder
ein pharmazeutisch annehmbares Salz, Hydrat und/oder Solvat davon,
mit der Maßgabe, dass wenigstens eines von R¹, R² und R⁴ ausgewählt ist aus der Gruppe, die Halogenalkyl, Halogenalkoxy, Heterocyclus, substituierten Heterocyclus, -NH₂, -NH(C₁-C₆-Alkyl) und -N(C₁-C₆-Alkyl)₂ umfasst.

11. Eine Verbindung der Formel (I) zur Verwendung bei der Prävention und/oder Linderung von Ischämie, Reperfusionsverletzung, Hirngefäßerkrankung, Lungengefäßerkrankung, Atherosklerose, Schlaganfall, Myokardinfarkt, Arterienerkrankung, kongestiver Herzerkrankung, Koronarerkrankung, peripherer vaskulärer Erkrankung, Diabetes, Hypertonie, Migräne, chronisch obstruktiver Lungenerkrankung oder retinaler Gefäßerkrankung: 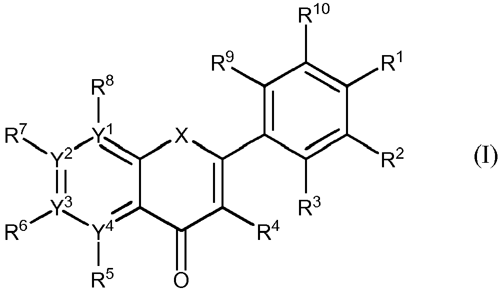 wobei
X NH, S oder O bedeutet,
Y¹, Y², Y³, Y⁴ unabhängig ausgewählt sind aus C oder N,
R¹, R² und R⁴ unabhängig ausgewählt sind aus der Gruppe, die Hydroxyl, Halogenalkyl, Halogenalkoxy, Heterocyclus, substituierten Heterocyclus, -NH₂, -NH(C₁-C₆-Alkyl) und -N(C₁-C₆-Alkyl)₂ umfasst,
R³, R⁹ und R¹⁰ unabhängig ausgewählt sind aus der Gruppe, die H, Halogen und C₁-C₆-Alkyl umfasst,
R⁵, R⁶, R⁷ und R⁸, wo vorhanden, H sind, oder
ein pharmazeutisch annehmbares Salz, Hydrat und/oder Solvat davon,
mit der Maßgabe, dass wenigstens eines von R¹, R² und R⁴ ausgewählt ist aus der Gruppe, die Halogenalkyl, Halogenalkoxy, Heterocyclus, substituierten Heterocyclus, -NH₂, -NH(C₁-C₆-Alkyl) und -N(C₁-C₆-Alkyl)₂ umfasst.

12. Eine Verbindung gemäß Anspruch 9 oder Anspruch 10 zur Verwendung bei der Prävention und/oder Linderung einer Erkrankung, die durch Zellapoptose und/oder Zellnekrose hervorgerufen wird, bei einem Subjekt, und/oder zur Verwendung bei der Prävention und/oder Behandlung einer oder mehrerer Erkrankungen, die mit dem Vorhandensein reaktiver oxidativer Spezies (ROS) assoziiert ist/sind, bei einem Subjekt, zur Verwendung bei der Behandlung einer Reperfusionsverletzung als Folge eines akuten oder chronischen Zustands, wobei das Subjekt an einem mechanischen Trauma leidet, das von einer Quetschverletzung oder einer Operation herrührt; Ischämie und/oder Reperfusionsverletzung als Folge eines akuten oder chronischen Zustands; einer Erkrankung, die die Folge einer Gefäßoperation ist, oder einer Erkrankung, die die Folge eines Herz-Bypasses und/oder einer Transplantationsoperation ist.

13. Eine Verbindung der Formel (I) zur Verwendung bei der Prävention und/oder Linderung von Ischämie oder Reperfusionsverletzung: 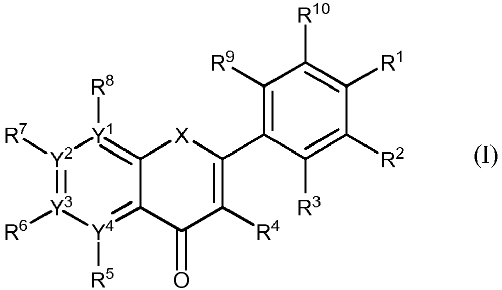 wobei
X NH, S oder O bedeutet,
Y¹, Y², Y³, Y⁴ unabhängig ausgewählt sind aus C oder N,
R¹, R² und R⁴ unabhängig ausgewählt sind aus der Gruppe, die Hydroxyl, Halogenalkyl, Halogenalkoxy, Heterocyclus, substituierten Heterocyclus, -NH₂, -NH(C₁-C₆-Alkyl) und -N(C₁-C₆-Alkyl)₂ umfasst,
R³, R⁹ und R¹⁰ unabhängig ausgewählt sind aus der Gruppe, die H, Halogen und C₁-C₆-Alkyl umfasst,
R⁵, R⁶, R⁷ und R⁸, wo vorhanden, H sind, oder
ein pharmazeutisch annehmbares Salz, Hydrat und/oder Solvat davon,
mit der Maßgabe, dass wenigstens eines von R¹, R² und R⁴ ausgewählt ist aus der Gruppe, die Halogenalkyl, Halogenalkoxy, Heterocyclus, substituierten Heterocyclus, -NH₂, -NH(C₁-C₆-Alkyl) und -N(C₁-C₆-Alkyl)₂ umfasst.

14. Eine Verbindung der Formel (I) zur Verwendung bei der Minderung eines Schadens bei einem Subjekt, der durch Verabreichung eines therapeutischen Mittels hervorgerufen wurde: 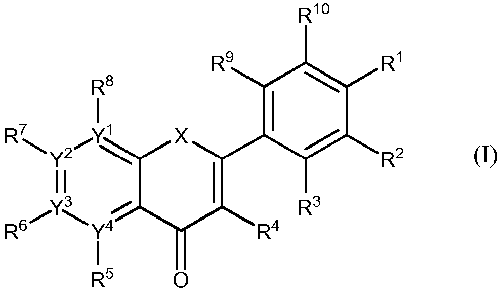 wobei
X NH, S oder O bedeutet,
Y¹, Y², Y³, Y⁴ unabhängig ausgewählt sind aus C oder N,
R¹, R² und R⁴ unabhängig ausgewählt sind aus der Gruppe, die Hydroxyl, Halogenalkyl, Halogenalkoxy, Heterocyclus, substituierten Heterocyclus, -NH₂, -NH(C₁-C₆-Alkyl) und -N(C₁-C₆-Alkyl)₂ umfasst,
R³, R⁹ und R¹⁰ unabhängig ausgewählt sind aus der Gruppe, die H, Halogen und C₁-C₆-Alkyl umfasst,
R⁵, R⁶, R⁷ und R⁸, wo vorhanden, H sind, oder
ein pharmazeutisch annehmbares Salz, Hydrat und/oder Solvat davon,
mit der Maßgabe, dass wenigstens eines von R¹, R² und R⁴ ausgewählt ist aus der Gruppe, die Halogenalkyl, Halogenalkoxy, Heterocyclus, substituierten Heterocyclus, -NH₂, -NH(C₁-C₆-Alkyl) und -N(C₁-C₆-Alkyl)₂ umfasst.

## Revendications

1. Composé de formule générale (I) : 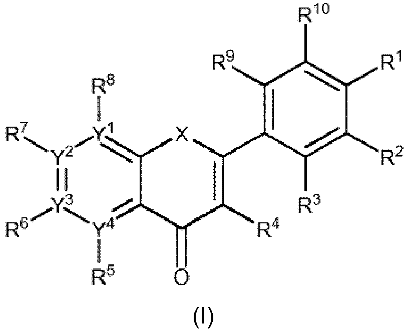 dans laquelle
X représente NH, S ou O ;
Y¹, Y², Y³ et Y⁴ sont indépendamment choisis parmi C et N ;
R¹, R² et R⁴ sont indépendamment choisis dans l'ensemble comprenant hydroxyle, halogénoalkyle, halogénoalcoxy, un hétérocyclique et un hétérocyclique substitué, -NH₂, -NH-alkyle en C₁ à C₆ et -N-(alkyle en C₁ à C₆)₂ ;
R³, R⁹ et R¹⁰ sont indépendamment choisis dans l'ensemble comprenant H, un halogène et un alkyle en C₁ à C₆ ;
R⁵, R⁶, R⁷ et R⁸, lorsqu'ils sont présents, sont H ; ou
un sel, hydrate et/ou solvate pharmaceutiquement acceptable d'un tel composé ;
sous réserve qu'au moins l'un de R¹, R² et R⁴ soit choisi dans l'ensemble comprenant halogénoalkyle, halogénoalcoxy, un hétérocyclique, un hétérocyclique substitué, -NH₂, -NH-alkyle en C₁ à C₆, et -N-(alkyle en C₁ à C₆)₂ ;
sous réserve que le composé ne soit pas choisi dans l'ensemble comprenant les suivants
2-(3,4-dihydroxyphényl)-3,5,7-trihydroxy-4H-1-benzothiopyran-4-one ;
2-(2,3-dihydroxyphényl)-5,8-dihydroxy-4H-1-benzothiopyran-4-one ;
2-(3,4-dihydroxyphényl)-3-hydroxy-4(1H)-quinolinone ;
bromhydrate de 2-(3,4-dihydroxyphényl)-3,7-dihydroxy-4(1H)-quinolinone ;
2-(3,4-dihydroxyphényl)-3,7-dihydroxy-4(1H)-quinolinone ;
2-(3,4-dihydroxyphényl)-3,6-dihydroxy-4(1H)-quinolinone ;
bromhydrate de 2-(3,4-dihydroxyphényl)-3,5-dihydroxy-4(1H)-quinolinone ;
2-(3,4-dihydroxyphényl)-3,5-dihydroxy-4(1H)-quinolinone ;
2-(3,4-dihydroxyphényl)-3,6,7-trihydroxy-4(1H)-quinolinone ;
2-(3,4-dihydroxyphényl)-3,5,7-trihydroxy-4(1H)-quinolinone ;
bromhydrate de 2-(3,4-dihydroxyphényl)-3-hydroxy-5,7-diméthyl-4(1H)-quinolinone ;
2-(3,4-dihydroxyphényl)-3-hydroxy-5,7-diméthyl-4(1H)-quinolinone ;
2-(3,4-dihydroxyphényl)-3-hydroxy-7-méthoxy-4(1H)-quinolinone ;
2-(3,4-dihydroxyphényl)-3-hydroxy-6-méthoxy-4(1H)-quinolinone ;
2-(3,4-dihydroxyphényl)-3,6,7,8-tétrahydroxy-4(1H)-quinolinone ;
2-(3,4-dihydroxyphényl)-3-hydroxy-5-méthoxy-4(1H)-quinolinone ;
2-(3,4-dihydroxyphényl)-3-hydroxy-6,7-diméthoxy-4(1H)-quinolinone ;
2-(3-amino-4-hydroxyphényl)-3,5,7-trihydroxy-4H-1-benzopyran-4-one ;
N-[2-(3-amino-4-hydroxyphényl)-3-hydroxy-6-méthyl-4-oxo-4H-chromén-8-yl]acétamide ;
N-[3-hydroxy-2-[4-hydroxy-3-[91-méthyléthyl)amino]phényl]6-méthyl-4-oxo-4H-1-benzopyran-8-yl]acétamide ;
N-[2-hydroxy-5-(3-hydroxy-6-méthyl-4-oxo-4H-1-benzopyran-2-yl)phényl]acétamide ;
2-(3-amino-4-hydroxyphényl)-3-hydroxy-6-méthyl-4H-1-benzopyran-4-one ;
8-amino-2-(3-amino-4-hydroxyphényl)-3-hydroxy-6-méthyl-4H-1-benzopyran-4-one ;
N-[2-hydroxy-5-(3-hydroxy-6-méthyl-4-oxo-4H-1-benzopyran-2-yl)phényl]acétamide ;
N-[2-(3,4-dihydroxyphényl)-3-hydroxy-4-oxo-4H-1-benzopyran-6-yl]acétamide ;
N-[2-(3-amino-4-hydroxyphényl)-3-hydroxy-6-méthyl-4-oxo-4H-1-benzopyran-8-yl]acétamide ;
6-acétamido-3-hydroxy-4'-méthoxyflavone ;
3,3'-dihydroxy,6,7-diméthyl,4'-(pyrrolidine-1-yl)flavone ; et
2-(3-amino-4-hydrophényl)-3,5,7-trihydroxy-4H-1-benzopyran-4-one.

2. Composé selon la revendication 1, dans lequel l'halogénoalkyle est choisi dans l'ensemble comprenant -CF₂H, -CH₂F, -CH₂CF₂H, et -CF₂CF₂H.

3. Composé selon la revendication 1 ou la revendication 2, dans lequel l'halogénoalcoxy est choisi dans l'ensemble comprenant -OCF₂H, -OCH₂F, -OCH₂CF₂H, et -OCF₂CF₂H.

4. Composé selon l'une quelconque des revendications précédentes, dans lequel l'hétérocyclique est choisi dans le groupe comprenant

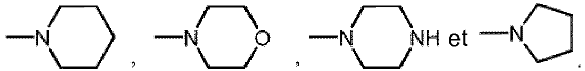

5. Composé selon l'une quelconque des revendications précédentes, dans lequel le groupe -NH-alkyle en C₁ à C₆ est choisi dans l'ensemble comprenant -NHCH₃, - NHCH₂CH₃ et -NHCH₂CH₂CH₃.

6. Composé selon l'une quelconque des revendications précédentes, dans lequel le groupe -N-(alkyle en C₁ à C₆)₂ est choisi parmi -N(CH₃)₂, -N(CH₂CH₃)₂, et - N(CH₂CH₂CH₃)₂.

7. Composé selon l'une quelconque des revendications précédentes, dans lequel :
X est O ;
Y¹, Y², Y³ et Y⁴ sont C ;
R⁵, R⁶, R⁷ et R⁸ sont H ; ou
X est O ;
Y¹, Y³ et Y⁴ sont C ;
Y² est N ;
R⁵, R⁶ et R⁸ sont H ;
R⁷ est absent ; ou
X est O ;
Y¹, Y² et Y⁴ sont C ;
Y³ est N ;
R⁵, R⁷ et R⁸ sont H ;
R⁶ est absent ; ou
X est NH ;
Y¹, Y², Y³ et Y⁴ sont C ;
R⁵, R⁶, R⁷ et R⁸ sont H ; ou
X est S ;
Y¹, Y², Y³ et Y⁴ sont C ;
R⁵, R⁶, R⁷ et R⁸ sont H ; et
R¹ est OH, R² est NH₂ et R⁴ est OH ; ou
R¹ est NH₂, R² est OH et R⁴ est OH ; ou
R¹ est OH, R² est NHCH₃ et R⁴ est OH ; ou
R¹ est NHCH₃, R² est OH et R⁴ est OH ; ou
R¹ est CF₂H, R² est OH et R⁴ est OH ; ou
R¹ est OH, R² est CF₂H et R⁴ est OH ; ou
R¹ est CF₂H, R² est CF₂H et R⁴ est OH ; ou
R¹ est OH, R² est OCF₂H et R⁴ est OH ; ou
R¹ est OCF₂H, R² est OH et R⁴ est OH ; ou
R¹ est OCF₂H, R² est OCF₂H et R⁴ est OH ; ou
R¹ est OCF₂H, R² est CF₂H et R⁴ est OH ; ou
R¹ est CF₂H, R² est OCF₂H et R⁴ est OH ; ou
R¹ est OH, R² est NH₂ et R⁴ est OH ; ou
R¹ est OH, R² est OH et R⁴ est NH₂ ; ou
R¹ est NH₂, R² est OH et R⁴ est NH₂ ; ou
R¹ est OH, R² est NHCH₃ et R⁴ est NH₂ ; ou
R¹ est NHCH₃, R² est OH et R⁴ est NH₂ ; ou
R¹ est CF₂H, R² est OH et R⁴ est NH₂ ; ou
R¹ est OH, R² est CF₂H et R⁴ est NH₂ ; ou
R¹ est CF₂H, R² est CF₂H et R⁴ est NH₂ ; ou
R¹ est OH, R² est OCF₂H et R⁴ est NH₂ ; ou
R¹ est OCF₂H, R² est OH et R⁴ est NH₂ ; ou
R¹ est OCF₂H, R² est OCF₂H et R⁴ est NH₂ ; ou
R¹ est OCF₂H, R² est CF₂H et R⁴ est NH₂ ; ou
R¹ est CF₂H, R² est OCF₂H et R⁴ est NH₂ ; ou
R¹ est OH, R² est OH et R⁴ est NHCH₃ ; ou
R¹ est OH, R² est NH₂ et R⁴ est NHCH₃ ; ou
R¹ est NH₂, R² est OH et R⁴ est NHCH₃ ; ou
R¹ est CF₂H, R² est OH et R⁴ est NHCH₃ ; ou
R¹ est OH, R² est CF₂H et R⁴ est NHCH₃ ; ou
R¹ est OCF₂H, R² est OH et R⁴ est NHCH₃ ; ou
R¹ est OH, R² est OCF₂H et R⁴ est NHCH₃ ; ou
R¹ est OH, R² est OH et R⁴ est NHCH₃ ; ou
R¹ est NH₂, R² est OH et R⁴ est NHCH₃ ; ou
R¹ est OH, R² est NHCH₃ et R⁴ est NHCH₃ ; ou
R¹ est NHCH₃, R² est OH et R⁴ est NHCH₃ ; ou
R¹ est CF₂H, R² est OH et R⁴ est NHCH₃ ; ou
R¹ est OH, R² est CF₂H et R⁴ est NHCH₃ ; ou
R¹ est CF₂H, R² est CF₂H et R⁴ est NHCH₃ ; ou
R¹ est OH, R² est OCF₂H et R⁴ est NHCH₃ ; ou
R¹ est OCF₂H, R² est OH et R⁴ est NHCH₃ ; ou
R¹ est OCF₂H, R² est OCF₂H et R⁴ est NHCH₃ ; ou
R¹ est OCF₂H, R² est CF₂H et R⁴ est NHCH₃ ; ou
R¹ est CF₂H, R² est OCF₂H et R⁴ est NHCH₃ ; ou
R¹ est OH, R² est 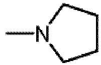 et R⁴ est OH ; ou
R¹ est 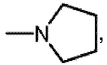 R² est OH et R⁴ est OH.

8. Composition pharmaceutique et/ou vétérinaire comprenant un véhicule ou diluant acceptable du point de vue pharmaceutique et/ou vétérinaire conjointement avec un composé selon l'une quelconque des revendications 1 à 7.

9. Composé de formule (I) pour une utilisation dans la prévention et/ou l'atténuation d'une maladie provoquée par une apoptose cellulaire et/ou une nécrose cellulaire chez un sujet ; 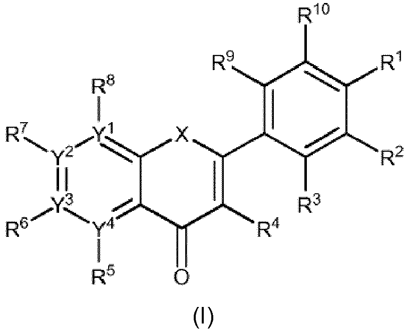 formule dans laquelle
X représente NH, S ou O ;
Y¹, Y², Y³ et Y⁴ sont indépendamment choisis parmi C et N ;
R¹, R² et R⁴ sont indépendamment choisis dans l'ensemble comprenant hydroxyle, halogénoalkyle, halogénoalcoxy, un hétérocyclique, un hétérocyclique substitué, -NH₂, -NH-alkyle en C₁ à C₆ et -N-(alkyle en C₁ à C₆)₂ ;
R³, R⁹ et R¹⁰ sont indépendamment choisis dans l'ensemble comprenant H, un halogène et un alkyle en C₁ à C₆ ;
R⁵, R⁶, R⁷ et R⁸, lorsqu'ils sont présents, sont H ; ou
un sel, hydrate et/ou solvate pharmaceutiquement acceptable d'un tel composé ;
sous réserve qu'au moins l'un de R¹, R² et R⁴ soit choisi dans l'ensemble comprenant halogénoalkyle, halogénoalcoxy, un hétérocyclique, un hétérocyclique substitué, -NH₂, -NH-alkyle en C₁ à C₆, et -N-(alkyle en C₁ à C₆)₂.

10. Composé de formule (I) pour une utilisation dans la prévention et/ou le traitement d'une ou plusieurs maladies chez un sujet associées à la présence d'espèces oxydatives réactives (ROS) : 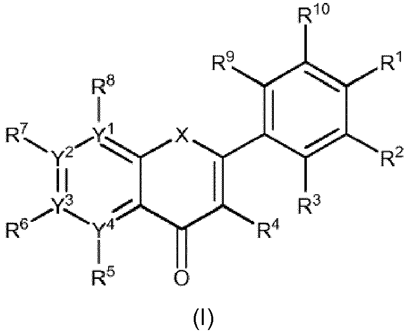 formule dans laquelle
X représente NH, S ou O ;
Y¹, Y², Y³ et Y⁴ sont indépendamment choisis parmi C et N ;
R¹, R² et R⁴ sont indépendamment choisis dans l'ensemble comprenant hydroxyle, halogénoalkyle, halogénoalcoxy, un hétérocyclique, un hétérocyclique substitué, -NH₂, -NH-alkyle en C₁ à C₆ et -N-(alkyle en C₁ à C₆)₂ ;
R³, R⁹ et R¹⁰ sont indépendamment choisis dans l'ensemble comprenant H, un halogène et un alkyle en C₁ à C₆ ;
R⁵, R⁶, R⁷ et R⁸, lorsqu'ils sont présents, sont H ; ou
un sel, hydrate et/ou solvate pharmaceutiquement acceptable d'un tel composé ;
sous réserve qu'au moins l'un de R¹, R² et R⁴ soit choisi dans l'ensemble comprenant halogénoalkyle, halogénoalcoxy, un hétérocyclique, un hétérocyclique substitué, -NH₂, -NH-alkyle en C₁ à C₆, et -N-(alkyle en C₁ à C₆)₂.

11. Composé de formule (I) pour une utilisation dans la prévention et/ou l'atténuation d'une ischémie, d'une lésion par reperfusion, d'une maladie cérébrovasculaire, d'une maladie vasculaire pulmonaire, de l'athérosclérose, d'un accident vasculaire, d'un infarctus du myocarde, d'une maladie artérielle, d'une maladie cardiaque congestive, d'une maladie coronarienne, d'une maladie vasculaire périphérique, du diabète, de l'hypertension, d'une migraine, d'une bronchopneumopathie chronique obstructive, ou d'une maladie vasculaire rétinienne : 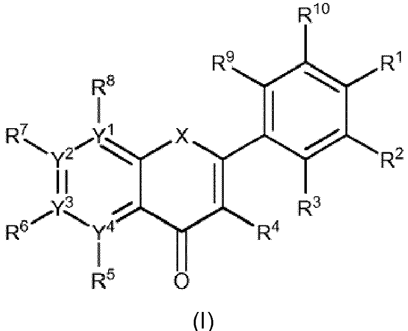 formule dans laquelle
X représente NH, S ou O ;
Y¹, Y², Y³ et Y⁴ sont indépendamment choisis parmi C et N ;
R¹, R² et R⁴ sont indépendamment choisis dans l'ensemble comprenant hydroxyle, halogénoalkyle, halogénoalcoxy, un hétérocyclique, un hétérocyclique substitué, -NH₂, -NH-alkyle en C₁ à C₆ et -N-(alkyle en C₁ à C₆)₂ ;
R³, R⁹ et R¹⁰ sont indépendamment choisis dans l'ensemble comprenant H, un halogène et un alkyle en C₁ à C₆ ;
R⁵, R⁶, R⁷ et R⁸, lorsqu'ils sont présents, sont H ; ou
un sel, hydrate et/ou solvate pharmaceutiquement acceptable d'un tel composé ;
sous réserve qu'au moins l'un de R¹, R² et R⁴ soit choisi dans l'ensemble comprenant halogénoalkyle, halogénoalcoxy, un hétérocyclique, un hétérocyclique substitué, -NH₂, -NH-alkyle en C₁ à C₆, et -N-(alkyle en C₁ à C₆)₂.

12. Composé selon la revendication 9 ou la revendication 10 pour une utilisation dans la prévention et/ou l'atténuation d'une maladie provoquée par une apoptose cellulaire et/ou une nécrose cellulaire chez un sujet, et/ou pour une utilisation dans la prévention et/ou le traitement d'une ou plusieurs maladies chez un sujet associées à la présence d'espèces oxydatives réactives (ROS) pour une utilisation dans le traitement d'une lésion par reperfusion en résultat d'un état aigu ou chronique, le sujet souffrant d'un traumatisme mécanique résultant d'une lésion par écrasement ou d'une chirurgie ; d'une ischémie et/ou d'une lésion par reperfusion en résultat d'un état aigu ou chronique ; d'une maladie qui est le résultat d'une chirurgie vasculaire ou d'une maladie qui est le résultat d'une dérivation cardiaque et/ou d'une greffe chirurgicale.

13. Composé de formule (I) pour une utilisation dans la prévention et/ou l'atténuation d'une ischémie ou d'une lésion par reperfusion : 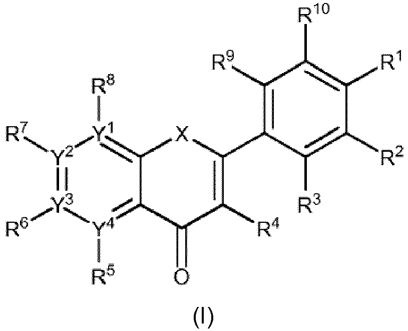 formule dans laquelle
X représente NH, S ou O ;
Y¹, Y², Y³ et Y⁴ sont indépendamment choisis parmi C et N ;
R¹, R² et R⁴ sont indépendamment choisis dans l'ensemble comprenant hydroxyle, halogénoalkyle, halogénoalcoxy, un hétérocyclique, un hétérocyclique substitué, -NH₂, -NH-alkyle en C₁ à C₆ et -N-(alkyle en C₁ à C₆)₂ ;
R³, R⁹ et R¹⁰ sont indépendamment choisis dans l'ensemble comprenant H, un halogène et un alkyle en C₁ à C₆ ;
R⁵, R⁶, R⁷ et R⁸, lorsqu'ils sont présents, sont H ; ou
un sel, hydrate et/ou solvate pharmaceutiquement acceptable d'un tel composé ;
sous réserve qu'au moins l'un de R¹, R² et R⁴ soit choisi dans l'ensemble comprenant halogénoalkyle, halogénoalcoxy, un hétérocyclique, un hétérocyclique substitué, -NH₂, -NH-alkyle en C₁ à C₆, et -N-(alkyle en C₁ à C₆)₂.

14. Composé de formule (I) pour une utilisation dans l'amélioration des dommages à un sujet provoqués par l'administration d'un agent thérapeutique : 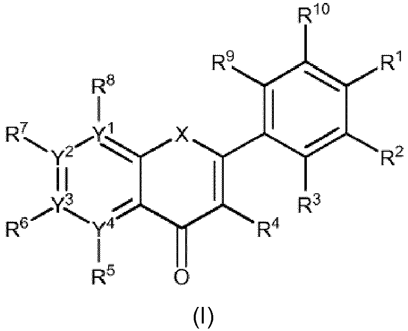 formule dans laquelle
X représente NH, S ou O ;
Y¹, Y², Y³ et Y⁴ sont indépendamment choisis parmi C et N ;
R¹, R² et R⁴ sont indépendamment choisis dans l'ensemble comprenant hydroxyle, halogénoalkyle, halogénoalcoxy, un hétérocyclique, un hétérocyclique substitué, -NH₂, -NH-alkyle en C₁ à C₆ et -N-(alkyle en C₁ à C₆)₂ ;
R³, R⁹ et R¹⁰ sont indépendamment choisis dans l'ensemble comprenant H, un halogène et un alkyle en C₁ à C₆ ;
R⁵, R⁶, R⁷ et R⁸, lorsqu'ils sont présents, sont H ; ou
un sel, hydrate et/ou solvate pharmaceutiquement acceptable d'un tel composé ;
sous réserve qu'au moins l'un de R¹, R² et R⁴ soit choisi dans l'ensemble comprenant halogénoalkyle, halogénoalcoxy, un hétérocyclique, un hétérocyclique substitué, -NH₂, -NH-alkyle en C₁ à C₆, et -N-(alkyle en C₁ à C₆)₂.
